(19) Europäisches Patentamt
European Patent Office
Office européen des brevets

(11) EP 1 736 472 A1

(12) **EUROPEAN PATENT APPLICATION**
published in accordance with Art. 158(3) EPC

(43) Date of publication:
**27.12.2006 Bulletin 2006/52**

(21) Application number: **05730482.6**

(22) Date of filing: **13.04.2005**

(51) Int Cl.:
*C07D 401/04* (2006.01) *A61K 31/506* (2006.01)
*A61K 31/55* (2006.01) *A61P 29/00* (2006.01)
*A61P 35/00* (2006.01) *A61P 37/06* (2006.01)
*C07D 401/14* (2006.01) *C07D 403/04* (2006.01)
*C07D 413/14* (2006.01)

(86) International application number:
**PCT/JP2005/007178**

(87) International publication number:
**WO 2005/100341 (27.10.2005 Gazette 2005/43)**

(84) Designated Contracting States:
**AT BE BG CH CY CZ DE DK EE ES FI FR GB GR HU IE IS IT LI LT LU MC NL PL PT RO SE SI SK TR**

(30) Priority: **15.04.2004 JP 2004120833**

(71) Applicant: **Astellas Pharma Inc.**
**Tokyo 103-8411 (JP)**

(72) Inventors:
- **OKAMOTO, Yoshinori, Astellas Pharma Inc. Tokyo 103-8411 (JP)**
- **KUBOTA, Hirokazu, Astellas Pharma Inc. Tokyo 103-8411 (JP)**
- **SATO, Ippei, Astellas Pharma Inc. Tokyo 103-8411 (JP)**
- **HATTORI, Kazuyuki, Astellas Pharma Inc. Tokyo 103-8411 (JP)**
- **KANAYAMA, Takatoshi, Astellas Pharma Inc. Tokyo 103-8411 (JP)**
- **YOKOYAMA, Kazuhiro, Astellas Pharma Inc. Tokyo 103-8411 (JP)**
- **TERAI, Yoshiya, Astellas Pharma Inc. Tokyo 103-8411 (JP)**
- **TAKEUCHI, Masahiro, Astellas Pharma Inc. Tokyo 103-8411 (JP)**

(74) Representative: **HOFFMANN EITLE**
**Patent- und Rechtsanwälte**
**Arabellastrasse 4**
**81925 München (DE)**

(54) **2-AMINOPYRIDINE DERIVATIVE**

(57) The invention relates to a compound useful as remedies or preventives for IKK2-related inflammatory diseases or autoimmune diseases, or that is, a 2-aminopyrimidine derivative having a 2-hydroxyphenyl group at the 6-position thereof and having a saturated cyclic group that contains one nitrogen atom, such as piperidine, at the 4-position thereof. The pharmaceutical composition of the invention and the compound of the invention have an excellent antiinflammatory effect based on the IKK2-inhibitory effect thereof, and are therefore useful for remedies and preventives for inflammatory diseases and autoimmune diseases, especially for rheumatoid arthritis.

EP 1 736 472 A1

## Description

TECHNICAL FIELD

The present invention relates to a 2-aminopyrimidine derivative useful for medicines, especially for remedies or preventives for IKK2-related diseases such as rheumatoid arthritis.

BACKGROUND ART

Nucleofactor κB (NF-κB) is a ubiquitous transcription factor which activates the transcription and translation of a protein that contributes to inflammatory reaction, such as cytokine (e.g., TNF-α, IL-1β, IL-6), chemokine (e.g., RANTES, IL-8) or arachidonic acid-metabolic enzyme (e.g., COX-2), and it plays an important role in acute inflammatory reaction and chronic inflammatory reaction in inflammatory diseases or autoimmune diseases (Mol. Cell Biol., 1999;19: 4547-51). For example, it is shown that, in the synovial cells of a case of rheumatoid arthritis (RA), NF-κB translocates to the nuclei and is activated therein, and it is pointed out that NF-κB plays a leading role in producing inflammatory mediators such as cytokine and eicosanoid in inflamed areas (Annu. Rev. Immunol., 1994;12:141-79).
It is shown that inhibiting the activation of NF-κB may be an effective therapeutical method for the above-mentioned inflammatory cases. For example, steroids, non-steroidal antiinflammatory drugs (NSAID; such as salicylate, sulindac), immunomodulators (e.g., thalidomide) or antioxidants (e.g., flavonoids) that are widely used in clinical sites have an effect of inhibiting the activation of NF-κB (Nat. Rev. Drug Discov., 2004;3:17-26).
In general, NF-κB stays in cytoplasm, binding to its repressor IκB therein. When cells are stimulated by a cytokine (e.g., TNF-α, IL-1β), a bacterial or viral product (e.g., LPS, TAX) or a chemical substance (e.g., phorbol ester), then IκB therein is phosphorylated by an IκB kinase (IKK1, IKK2). As a result, the active NF-κB released from IκB may translocate to the nuclei, in which it binds to a specific enhancer sequence and therefore starts the transcription and translation of cytokine, etc., as mentioned above.
It is suggested that IKK2 is important for activation of NF-κB, and that inhibiting IKK2 is a most effective method for selectively inhibiting the activation of NF-κB and inhibiting the inflammation based on it (Nat. Rev. Drug Discov., 2004; 3:17-26). In addition, heretofore it is shown that, in IKK2-deleted fibroblasts, NF-κB is not activated by cytokine attack (Science, 1999;284:321-5). Further, it is reported that, in various animal models, a low-molecular compound capable of selectively inhibiting IKK2 inhibits inflammatory reaction (Nat. Rev. Drug Discov., 2004;3:17-26); or in animals where an IKK2 mutant with no enzymatic activity is expressed, inflammatory reaction is inhibited (Arthritis Rheum., 2001; 44: 1897-907).
Recently, biological preparations of anti-cytokine antibody such as TNF-α or IL-1 have been specifically noted for therapy of inflammatory diseases or autoimmune diseases, but it is reported that, when the preparation is used alone, then its therapeutical effect is not sufficient (Nat. Rev. Drug Discov., 2003;2:473-88). In fact, in general, a biological preparation is used along with NSAID or steroid in clinical sites (Lancet, 1999;354:1932-9). On the other hand, in arthritic rat models, combined administration of an anti-TNF-α preparation and an anti-IL-1 preparation has brought about a synergistic therapeutical effect (Arthritis Rheum., 2001;43:2648-2659). Accordingly, there is a possibility that an IKK2 inhibitor capable of simultaneously inhibiting various inflammatory factors in addition to TNF-α and IL-1 may have a higher pharmaceutical potency than that of medicines currently used for therapy of acute and chronic inflammations and autoimmune diseases.
It is reported that IKK2 participates in expression control of anti-apoptosis protein (e.g., Bcl-2) and IKK2 inhibitors show antitumor effects (Drug Discovery Today 2002;7:653-63).

It is suggested that a pyridine derivative of the following general formula (II) has an IKK2-inhibitory effect and is effective for therapy of asthma or arthritis (Patent Reference 1):

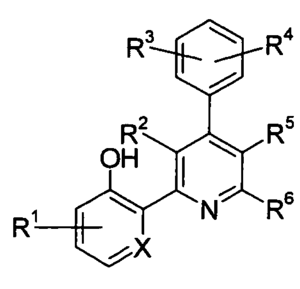

(II)

(wherein X represents CH or N; $R^1$ represents hydrogen atom, hydroxy or halogen, etc.; $R^2$ represents hydrogen atom, etc.; $R^3$ represents hydrogen atom, hydroxy or halogen, etc.; $R^4$ represents hydrogen atom, hydroxy or carboxy, etc.;

$R^5$ represents hydrogen atom or cyano, etc.; $R^6$ represents $NR^{61}R^{62}$ [$R^{61}$ represents hydrogen atom or $C_{1-6}$-alkyl; $R^{62}$ represents H, $C_{1-6}$-alkyl or Ph, etc.]; for their details, referred to is the published application).

It is disclosed that a pyridine derivative of the following general formula (III) has an IKK2-inhibitory effect and is effective for therapy of asthma or arthritis (Patent Reference 2):

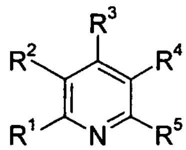 (III) R1: 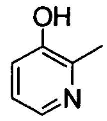 , 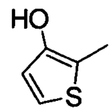 , 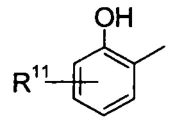

(wherein $R^2$ represents hydrogen atom or halogen; $R^3$ represents $-CR^{31}R^{32}R^{33}$, etc. [$R^{31}$ represents hydrogen atom or $C_{1-6}$-alkyl; $R^{32}$ and $R^{33}$ may form, along with the carbon atom thereof, a substitutable 5- to 8-membered saturated hetero ring containing from 0 to 3 hetero atoms selected from N, O and S]; $R^4$ represents hydroxycarbonyl, $C_{1-6}$-alkanoyl or cyano, etc.; $R^5$ represents $NR^{51}R^{52}$ [$R^{51}$ represents H or $C_{1-6}$-alkyl; $R^{52}$ represents H, $C_{1-6}$-alkyl or Ph, etc.]; $R^{11}$ represents hydrogen atom, etc.; for their details, referred to is the published application).

The corresponding Japanese patent application that is the basis of the priority of Patent Reference 2 describes a phenol group-substituted pyridine derivative (Patent Reference 3).

On the other hand, it is suggested that a pyrimidine derivative of the following general formula (IV) has a dopamine-modulating effect and is effective for therapy of central, digestive or cardiovascular diseases (Patent Reference 4). However, the published reference has no description relating to IKK2-inhibitory effect.

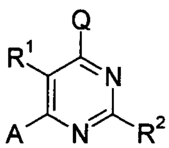 (IV) A: 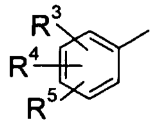 , 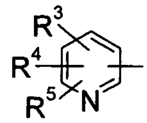 , 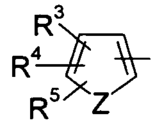

(wherein $R^1$ represents hydrogen atom or halogen, etc.; Q represents a 5- or 6-membered monocyclic saturated hetero ring substituted with one substituent and having one nitrogen atom as the hetero atom thereof, which bonds to the pyrimidine ring via its carbon atom; $R^2$ represents $-NR^aR^b$ [$R^a$ and $R^b$ each independently represent hydrogen atom or hydrocarbon group, etc.], etc.; $R^3$, $R^4$ and $R^5$ each independently represent hydrogen atom, hydrocarbon group or $-OR^a$, etc.; for their details, referred to is the published reference).

Patent References 5 and 6 report a pyrimidine derivative of the following general formula (V). Patent Reference 5 suggests that the derivative has a TNF-$\alpha$ production-inhibitory effect and is effective for therapy of HIV, asthma or ARDS, etc.; and Patent Reference 6 suggests that it is effective for therapy of rheumatoid arthritis. However, the published references have no description relating to IKK2-inhibitory effect.

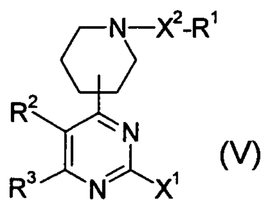 (V)

(wherein $R^3$ represents alkyl, substituted or unsubstituted aryl group, etc.; $R^2$ represents hydrogen atom or alkyl group, etc.; $X^2$ represents carbonyl, carbonyloxy, carbonylamino or sulfonyl group; $R^1$ represents alkyl, cycloalkyl, substituted or unsubstituted aryl, etc.; $X^1$ represents amino group or hydroxyl group; for their details, referred to is the published references).

Patent Reference 1: WO02/044153
Patent Reference 2: WO02/024679
Patent Reference 3: JP-A 2002-114777
Patent Reference 4: USP 5763448

Patent Reference 5: USP 5948786
Patent Reference 6: JP-A 2003-095951

DISCLOSURE OF THE INVENTION

PROBLEMS THAT THE INVENTION IS TO SOLVE

A subject matter of the present invention is to provide a pharmaceutical composition having a strong antiinflammatory effect based on its IKK2-inhibitory effect and therefore effective for remedy or prevention of inflammatory diseases or autoimmune diseases such as rheumatoid arthritis.

MEANS FOR SOLVING THE PROBLEMS

We, the present inventors have assiduously studied compounds having an IKK2-inhibitory effect and, as a result, have confirmed that a 2-aminopyrimidine derivative represented by the following general formula, which has a 2-hydroxyphenyl group at the 6-position thereof and has a saturated cyclic group having one nitrogen atom such as piperidine, at the 4-position thereof, has an excellent antiinflammatory effect based on its IKK2-inhibitory effect, and have found that a pharmaceutical composition containing it as the active ingredient thereof could be a good remedy or preventive for inflammatory diseases or autoimmune diseases. On the basis of these findings, we have completed the present invention.
The compound serving as the active ingredient of the medicine of the invention differs from the compounds described in Patent references 1 to 3 in point of their structures in that the former has a pyrimidine ring as the basic nucleus thereof and that the pyrimidine ring in the former does not have a functional group such as cyano group. In addition, it differs from the compounds concretely described in Patent References 4 to 6 in point of their structures in that, in the former, a 2-hydroxyphenyl group directly bonds to the 6-position of the pyrimidine ring
Specifically, the invention relates to an IKK2-inhibitory agent containing, as the active ingredient thereof, a 2-aminopyrimidine derivative of the following general formula (I) or its salt
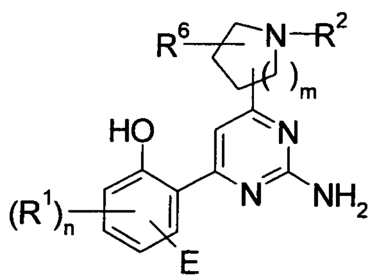

(I)

(wherein the symbols have the following meanings:

$R^1$: same or different from each other, each represents lower alkyl, -OH, -O-lower alkyl, halogen, halogeno lower alkyl, -S-$R^3$, -SO-$R^3$, -$SO_2$-$R^3$, -N$R^4R^5$), -$CO_2$-$R^3$,
-CO-N$R^4$($R^5$), -N$R^4$-CO-$R^0$, -CN, -$NO_2$, -O-halogeno lower alkyl or lower alkenylene; in these, lower alkyl and -O-lower alkyl may be substituted with one or two substituents selected from a group consisting of -O-$R^3$, -N$R^4$($R^5$), -CN and -$CO_2$-$R^3$;
$R^0$: lower alkyl;
$R^{00}$: lower alkylene;
$R^3$, $R^4$ and $R^5$: same or different from each other, these represent H or -$R^0$;
n: 0, 1 or 2;
E: H, -$E^1$ or -D-$E^1$;
$E^1$: optionally substituted cycloalkyl, optionally substituted phenyl, or optionally substituted monocyclic or bicyclic heterocyclic group;
D: bond, -O-, -S-, -$R^{00}$-, -O-$R^{00}$-, -$R^{00}$-O-, -N$R^4$-$R^{00}$-, -$R^{00}$-N$R^4$-, -N$R^4$-CO-, -CO-N$R^4$-, -O-$R^{00}$-N$R^4$-$R^{00}$-, -O-$R^{00}$-O-, -O-$R^{00}$-CO- or -O-$R^{00}$-CH(O-$R^3$)-;
$R^6$: H, -$R^{00}$-optionally substituted phenyl, or -$R^0$;
m: 0, 1, 2 or 3;

$R^2$: H, $-R^0$ or -Z-W;
Z: $-R^{00}-$, -CO-, $-CO-R^{00}-$ or $-R^{00}-CO-$;
W: $-O-R^3$, $-NR^4R^5$), $-CR^{21}R^{22}R^{23}$, optionally substituted phenyl, or optionally substituted monocyclic or bicyclic heterocyclic group;
$R^{21}$: H or $-R^0$;
$R^{22}$: $-R^0$, $-O-R^3$ or $-NR^4(R^5)$;
$R^{23}$: $-R^0$ or optionally substituted phenyl - the same shall apply hereinunder).

The active ingredient of formula (I) in the medicine of the invention has an IKK2-inhibitory effect; and differs from the compounds in Patent Reference 4 that are characterized by having a dopamine-modulating effect, or the compounds described in Patent References 5 and 6 that are characterized by their TNF-α production-inhibitory effect, in point of their functions and effects.

Further, the invention also relates to a novel 2-aminopyrimidine derivative of the following general formula (I') or its salt, which has an IKK2-inhibitory effect and is useful for a remedy or preventive for inflammatory diseases or autoimmune diseases. The compound of formula (I') differs from the compounds described in Patent References 4 to 6 in point of their structures, in that in the former, Z is $-R^{00}-CO-$ when m is 1 or 2.

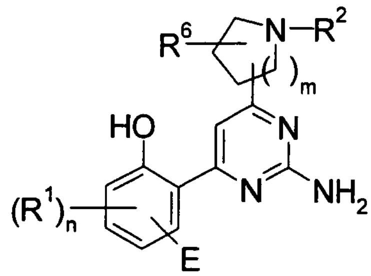

(I')

(wherein the symbols have the following meanings:

$R^1$: same or different from each other, each represents lower alkyl, -OH, -O-lower alkyl, halogen, halogeno lower alkyl, $-S-R^3$, $-SO-R^3$, $-SO_2-R^3$, $-NR^4(R^5)$, $-CO_2-R^3$, $-CO-NR^4(R^5)$, $-NR^4-CO-R^0$, -CN, $-NO_2$, -O-halogeno lower alkyl or lower alkenylene;
in these, lower alkyl and -O-lower alkyl may be substituted with one or two substituents selected from a group consisting of $-O-R^3$, $-NR^4(R^5)$, -CN and $-CO_2-R^3$;
$R^0$: lower alkyl;
$R^{00}$: lower alkylene;
$R^3$, $R^4$ and $R^5$: same or different from each other, these represent H or $-R^0$;
n: 0, 1 or 2;
E: H, $-E^1$ or $-D-E^1$;
$E^1$: optionally substituted cycloalkyl, optionally substituted phenyl, or optionally substituted monocyclic or bicyclic heterocyclic group;
D: bond, -O-, -S-, $-R^{00}-$, $-O-R^{00}-$, $-R^{00}-O-$, $-NR^4-R^{00}-$, $-R^{00}-NR^4-$, $-NR^4-CO-$, $-CO-NR^4-$, $-O-R^{00}-NR^4-R^{00}-$, $-O-R^{00}-O-$, $-O-R^{00}-CO-$ or $-O-R^{00}-CH(O-R^3)-$;
$R^6$: H, $-R^{00}$-optionally substituted phenyl, or $-R^0$;
m: 0, 1, 2 or 3;
$R^2$: H, $-R^0$ or -Z-W;
Z: $-R^{00}-$, -CO-, $-CO-R^{00}-$ or $-R^{00}-CO-$, provided that, when m is 1 or 2, then Z is $-R^{00}-CO-$;
W: $-O-R^3$, $-NR^4(R^5)$, $-CR^{21}R^{22}R^{23}$, optionally substituted phenyl, or optionally substituted monocyclic or bicyclic heterocyclic group;
$R^{21}$: H or $-R^0$;
$R^{22}$: $-R^0$, $-O-R^3$ or $-NR^4(R^5)$;
$R^{23}$: $-R^0$ or optionally substituted phenyl - the same shall apply hereinunder).

The invention also relates to a pharmaceutical composition comprising a 2-aminopyrimidine derivative of formula (I') or its salt and a pharmaceutically-acceptable carrier. Preferably, the pharmaceutical composition is an IKK2-inhibitory agent, more preferably a preventive or remedy for inflammatory diseases or autoimmune diseases, even more preferably

for rheumatoid arthritis.
In another embodiment thereof, the invention includes use of a 2-aminopyrimidine derivative of formula (I') or its salt for production of a remedy for rheumatoid arthritis, and includes a method for prevention or remedy of rheumatoid arthritis that comprises administering an effective dose of a 2-aminopyrimidine derivative or its salt to mammals.

EFFECT OF THE INVENTION

The active ingredient of the medicine of the invention or the compound of the invention inhibits IKK2 that participates in various cytokine production, and therefore has the advantage of exhibiting an excellent antiinflammatory effect in models of inflammatory diseases or autoimmune diseases.

BEST MODE FOR CARRYING OUT THE INVENTION

The invention is described in detail hereinunder.
In the definitions of general formulae in this description, the term "lower" means a linear or branched carbon chain having from 1 to 6 carbon atoms (this is hereinafter abbreviated to $C_{1-6}$), unless otherwise specifically indicated. Accordingly, "lower alkyl" is $C_{1-6}$-alkyl, preferably $C_{1-4}$-alkyl, more preferably linear alkyl such as methyl, ethyl and propyl groups, and branched alkyl such as isopropyl butyl, isobutyl and tert-butyl groups. Especially preferred are methyl, ethyl, propyl and isopropyl groups.
"Lower alkylene" is $C_{1-6}$-alkylene, preferably $C_{1-4}$-alkylene, more preferably linear alkylene such as methylene, ethylene, propylene and butylene groups, or branched alkylene such as methylmethylene group. Especially preferred are methylene, trimethylene and tetramethylene groups.
"Halogen" means F, Cl, Br and I. "Halogeno lower alkyl" preferably means $C_{1-6}$-alkyl substituted with at least one halogen, more preferably $C_{1-6}$-alkyl substituted with from 1 to 5 F's, even more preferably fluoromethyl, difluoromethyl, trifluoromethyl, trifluoroethyl, tetrafluoroethyl and pentafluoroethyl groups.
"Cycloalkyl" is a $C_{3-10}$-cyclic saturated hydrocarbon group, and this may be optionally bridged. Preferably, it is monocyclic cycloalkyl, more preferably cyclopropyl, cyclobutyl, cyclopentyl, cyclohexyl, cycloheptyl and adamantyl groups.
"Monocyclic heterocyclic group" is a monocyclic, 3- to 8-membered, preferably 5- to 7-membered cyclic group having from 1 to 4 hetero atoms selected from O, S and N, and includes monocyclic heteroaryl which is unsaturated ring, monocyclic heterocycloalkyl which is saturated ring, and partially-hydrogenated monocyclic heteroaryl. Monocyclic heteroaryl preferably includes pyridyl, pyrazinyl, pyrimidinyl, pyridazinyl, imidazolyl, pyrrolyl, triazolyl, tetrazolyl, thienyl, furyl, thiazolyl, pyrazolyl, isothiazolyl, oxazolyl, isoxazolyl, thiadiazolyl and oxadiazolyl groups. Monocyclic heterocycloalkyl and partially-hydrogenated cyclic heteroaryl preferably include piperidyl, pyrrolidinyl, piperazinyl, azepanyl, diazepanyl, tetrahydrofuranyl, tetrahydropyranyl, morpholinyl and thiomorpholinyl groups.
"Bicyclic heterocyclic group" is a cyclic group derived from condensation of the above-mentioned monocyclic heterocycles or condensation of benzene ring and monocyclic heterocycle, preferably including indolyl, benzofuranyl, benzothienyl, indazolyl, benzothiazolyl, benzoxazolyl, quinolyl, isoquinolyl, quinazolyl, quinoxalinyl, dihydrobenzofuranyl, tetrahydroquinolyl and indolinyl groups.
In the "monocyclic heterocyclic group" and the "bicyclic heterocyclic group", the ring atom, S or N may be oxidized to form an oxide or dioxide. In the heterocycloalkyl or partially-hydrogenated heteroaryl group, any carbon atom may be substituted with an oxo group.
"Monocyclic or bicyclic heterocyclic group" for the group $E^1$ and "monocyclic heterocyclic group" for the group $E^2$ mentioned below preferably include tetrahydrofuranyl, pyrrolidinyl, piperidinyl, pyridyl, oxadiazolyl.
"Optionally substituted" is meant to indicate "unsubstituted" or "having the same or different, 1 to 5 substituents". For example, the substituent in "optionally substituted phenyl" and "optionally substituted monocyclic or bicyclic heterocyclic group" preferably includes $R^0$, -O-$R^3$, halogen, halogeno lower alkyl, -O-halogeno lower alkyl, -$CO_2$-$R^3$, -$NR^4$($R^5$), -N($R^4$)-CO-$R^3$, -N($R^4$)-$SO_2$-$R^3$, -$CONR^4$($R^5$), -$CO_2$-$R^0$, -CN, -$NO_2$, phenyl, benzyl. The substituent in "optionally substituted cycloalkyl" preferably includes $R^0$, -O-$R^3$, -$NR^4$($R^5$), oxo, -$CO_2$-$R^3$.
One preferred embodiment of the compound serving as the active ingredient of the medicine of the invention is a 2-aminopyrimidine derivative of formula (I') or its salt, more preferably, the following derivatives and their salts.

(1) Derivative of formula (I') where $R^2$ is H.
(2) More preferably, derivatives of the above (1) where m is 1 or 2, especially 1.
(3) More preferably, derivatives of the above (2) where E is H, or 2-aminopyrimidine derivatives of the following general formula (I"):

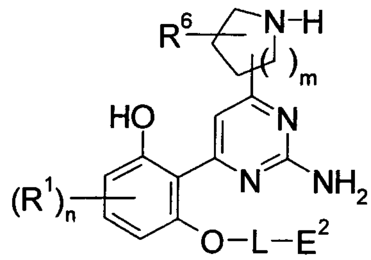

(I")

(wherein the symbols have the following meanings:

$R^1$: same or different from each other, each represents lower alkyl, -OH, -O-lower alkyl, halogen, halogeno lower alkyl, -S-$R^3$, -SO-$R^3$, -$SO_2$-$R^3$, -$NR^4(R^5)$, -$CO_2$-$R^3$,- CO-$NR^4(R^5)$, -$NR^4$-CO-$R^0$, -CN, -$NO_2$, -O-halogeno lower alkyl or lower alkenylene;
in these, lower alkyl and -O-lower alkyl may be substituted with one or two substituents selected from a group consisting of -O-$R^3$, -$NR^4(R^5)$, -CN and -$CO_2$-$R^3$;
$R^0$: lower alkyl;
$R^{00}$: lower alkylene;
$R^3$, $R^4$ and $R^5$: same or different from each other, these represent H or -$R^0$;
n: 0, 1 or 2;
$R^6$: H or -$R^0$;
m: 1 or 2;
L: bond, lower alkylene or lower alkylene-O-; in these, lower alkylene may be substituted with any of from 1 to 5 halogen, 1 or 2 -OH or oxo group;
$E^2$: optionally substituted cycloalkyl, optionally substituted phenyl, optionally substituted monocyclic heterocyclic group).

(4) More preferably, derivatives of the above (3) where $R^6$ is H.
(5) More preferably, derivatives of above (4) where $R^1$ is -$R^0$, -O-$R^3$, -S-$R^3$, halogen or halogeno lower alkyl, even more preferably methyl, ethyl, -OH, methoxy, ethoxy, methylthio or halogen.
(6) More preferably, derivatives of the above (3) where n is 0 or 1.
(7) Even more preferably, derivatives of formula (I') or their salts selected from the following group: 2-(2-Amino-6-piperidin-3-ylpyrimidin-4-yl)-4-methylphenol, 2-(2-amino-6-piperidin-3-ylpyrimidin-4-yl)-3-isobutoxyphenol, 2-(2-amino-6-piperidin-3-ylpyrimidin-4-yl)-3-(cyclobutylinethoxy)phenol,2-(2-amino-6-piperidin-3-ylpyrimidin-4-yl)-3-(cyclopentylmethoxy)phenol, 2-(2-amino-6-piperidin-3-ylpyrimidin-4-yl)-3-[(4-bromobenzyl)oxy]phenol, (+)-2-(2-amino-6-piperidin-3-ylpyrimidin-4-yl)-4-ethylphenol, (-)-2-(2-amino-6-piperidin-3-ylpyrimidin-4-yl)-4-ethylphenol, 2-(2-amino-6-piperidin-3-ylpyrimidin-4-yl)-3-[(2-methylcyclopropyl)methoxy]phenol, 2-(2-amino-6-piperidin-3-ylpyrimidin-4-yl)-3-[(1-methylcyclopropyl)methoxy]phenol, 2-(2-amino-6-piperidin-3-ylpyrimidin-4-yl)-3-(2-phenoxypropoxy)phenol, 2-(2-amino-6-piperidin-3-ylpyrimidin-4-yl)-3-(2,2,3,3-tetrapropoxy)phenol, 2-(2-amino-6-piperidin-3-ylpyrimidin-4-yl)-3-(2-cyclopropylethoxy)phenol, (+)-2-(2-amino-6-piperidin-3-ylpyrimidin-4-yl)-3-(cyclopropylmethoxy)phenol, (-)-2-(2-amino-6-piperidin-3-ylpyrimidin-4-yl)-3-(cyclopropylmethoxy)phenol, 2-(2-amino-6-(6-methylpiperidin-3-yl)pyrimidin-4-yl)-3-(cyclopropylmethoxy)phenol, 2-(2-amino-6-piperidin-4-ylpyrimidin-4-yl)benzene-1,3-diol, 2-(2-amino-6-piperidin-4-ylpyrimidin-4-yl)-3-(cyclopropylmethoxy)phenol, 2-(2-amino-6-piperidin-4-ylpyrimidin-4-yl)-3-isobutoxyphenol and 2-[2-(2-amino-6-piperidin-4-ylpyrimidin-4-yl)-3-hydroxyphenoxy]-1-phenylethanone.

Other preferred embodiments of the compound of formula (I) serving as the active ingredient of the medicine of the invention (hereinafter abbreviated as compound (I)), and the compound of formula (I') of the invention (hereinafter abbreviated as compound (I')) are the following compounds and their salts.
Compounds where $R^1$ is the same or different from each other and is -$R^0$, -O-$R^3$, halogen, halogeno lower alkyl, -S-$R^3$, -SO-$R^3$, -$SO_2$-$R^3$, -$NR^4(R^5)$, -$CO_2$-$R^3$, -CO-$NR^4(R^5)$ -$NR^4$-CO-$R^0$, -CN, -$R^{00}$-O-$R^3$, -$NO_2$ or -O-$R^{00}$-$CO_2$-$R^3$; D is bond, -O-, -S-, -$R^{00}$-, -O-$R^{00}$-, -$R^{00}$-O-, -$NR^4$-$R^{00}$-, -$R^{00}$-$NR^4$-, -$NR^4$-CO- or -CO-$NR^4$-; and $R^6$ is H.

Compounds (I) and (I') may have geometric isomers and tautomeric isomers. The invention includes those isomers that are isolated or combined.
Compounds (I) and (I') may have an asymmetric carbon atom, and may have optical isomers based on it. The invention includes all those optical isomers that are isolated or combined.

Further, compounds (I) and (I') include pharmaceutically-acceptable prodrugs. The pharmaceutically-acceptable prodrug is a compound having a group capable of being converted into $NH_2$, OH, $CO_2H$ or the like as in the invention, through solvolysis or under a physiological condition. Groups to form prodrugs are described in Prog. Med., 5, 2157-2161 (1985) ; Development of Medicines (by Hirokawa Publishing, 1990), Vol. 7, Molecular Design, 163-198.

Salts of compound (I) or (I') are pharmaceutically-acceptable salts, concretely including acid-addition salts with an inorganic acid such as hydrochloric acid, hydrobromic acid, hydroiodic acid, sulfuric acid, nitric acid, phosphoric acid; or an organic acid such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, aspartic acid, glutamic acid. Depending on the type of the substituent therein, the compounds may form salts with a base, including, for example, salts with an inorganic base that contains a metal such as sodium, potassium magnesium, calcium or aluminium, or with an organic base such as methylamine, ethylamine, ethanolamine, lysine or ornithine; and ammonium salts.

Further, compounds (I) and (I') and their salts include various hydrates, solvates and polymorphic crystalline substances.

(Production Methods)

The compound (I) and its pharmaceutically-acceptable salt serving as the active ingredient in the invention can be produced in various known synthetic methods, taking advantage of the characteristics based on the basic structure thereof or on the type of the substituent therein. Depending on the type thereof, the functional group in the starting material or in the intermediate may be protected with a suitable protective group, or may be substituted with a group readily convertible into the functional group. This is often effective in point of the production technique for the compounds. The functional group includes, for example, amino group, hydroxyl group, carboxyl group; and their protective groups are described, for example, in T. W. Greene and P. G. M. Wuts; Protective Groups in Organic Synthesis, 3rd Ed., 1999. Depending on the reaction condition, these may be suitably selected and used herein. In such a method, the reaction is attained after a protective group is introduced into the starting compound, and then optionally the protective group may be removed or may be converted into a desired group to thereby obtain the intended compound.

A prodrug of the compound may be produced by introducing a specific group into the obtained compound (I) or into the starting material or the intermediate. The reaction may be attained in any method known to those skilled in the art, for example, through esterification, amidation or dehydration.

Compounds (I') are all within the scope of compounds (I), and therefore, methods for producing compounds (I) are described below.

First Method:

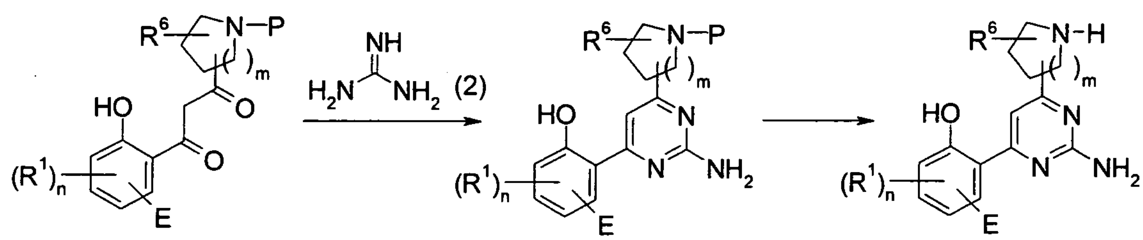

(2)

(1) (3) (Ia)

(I) $P=R^2$

(wherein P represents a protective group for amino group, or $R^2$ (but excepting H) - the same shall apply hereinunder). This method is for producing a compound (I) of the invention by cyclization reaction of a diketone derivative (1) with a guanidine (2). In particular, compounds (Ia) of falling within the scope of the compounds of the invention where $R^2$ is H may be produced by cyclization reaction of a compound having an amino-protective group at the site of $R^2$ in the manner as above, and then removing the protective group.

The cyclization may be attained by stirring a compound (1) and an equimolar amount of guanidine (2) or an excessive amount of guanidine (2), in a solvent inert to the reaction, at room temperature or under heat for reflux, generally for 1 hour to 3 days. The solvent usable herein includes alcohols such as methanol, ethanol, 2-propanol, butanol; ethers such as diethyl ether, tetrahydrofuran (THF), 1,4-dioxane, 1,2-dimethoxyethane, 1,2-diethoxyethane; halogenohydrocarbons such as dichloromethane, 1,2-dichloroethane, chloroform; N,N-dimethylformamide (DMF), N-methylpyrrolidone (NMP), dimethylsulfoxide (DMSO), pyridine, lutidine. However, not limited to these, herein usable is any solvent inert to the reaction.
For removing the protective group from the compound having an amino-protective group, usable are the methods described in Protective Groups in Organic Synthesis, 3rd Ed.

(Second Method)

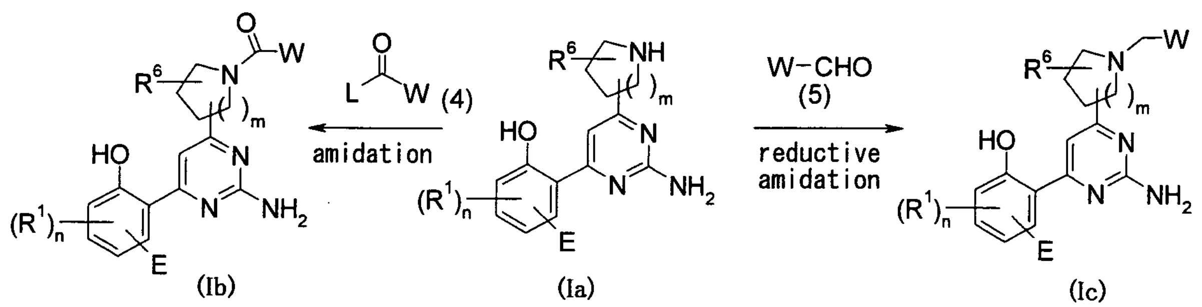


(Ib) (Ia) (Ic)

(wherein L represents a leaving group such as halogen, or -OH - the same shall apply hereinunder).
Of the compounds of formula (I), those having acyl group or alkyl group on the nitrogen atom of the nitrogen-containing saturated ring can be produced, for example, according to the following reaction.

(1) Amidation:

Compound (Ib) can be produced through amidation of a compound (Ia) obtained in the first method with a carboxylic acid or its reactive derivative (4). In case where a free carboxylic acid of compound (4) where L is OH is used, then a method may be employed that comprises condensing a compound (Ia) with the carboxylic acid (4) through dehydration in the presence of a condensing agent. For the condensing agent and the reaction condition in this method, for example, referred to are the methods described in Jikken Kagaku Koza (Courses in Experimental Chemistry), (4th Ed.), Vol. 22 by the Chemical Society of Japan (1992, by Maruzen). In case where a compound (4) where L is a leaving group, or that is, a reactive derivative of a carboxylic acid is used, then a compound (Ia) may be reacted with the reactive derivative (4) in the presence or absence of a base. For the base and the reaction condition in this method, for example, referred to are the methods described in Courses in Experimental Chemistry (4th Ed.), Vol. 22 by the Chemical Society of Japan (1992, by Maruzen).

(2) Reductive Amination:

Compound (Ic) may be produced through reductive amination of the compound (Ia) obtained in the first method with an aldehyde derivative (5). For the reaction, for example, referred to are the methods described in Courses in Experimental Chemistry (4th Ed.), Vol. 20 by the Chemical Society of Japan (1992, by Maruzen).

(Third Method)

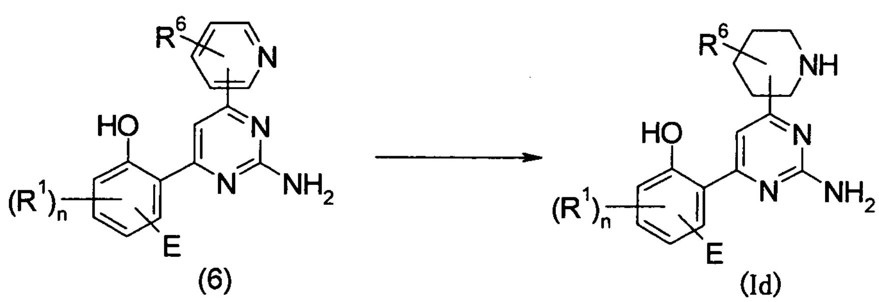

(6) (Id)

Of the compounds of formula (I), compounds (Id) where m is 2, and $R^2$ is H may be produced through reduction of a pyridine derivative (6).
The reduction may be attained by stirring a pyridine derivative (6) and any of various metal catalysts in a solvent inert to the reaction in a hydrogen atmosphere. For the metal catalyst and the reaction condition for the method, for example, referred to are the methods described in Courses in Experimental Chemistry (4th Ed.), Vol. 26 by the Chemical Society of Japan (1992, by Maruzen).

(Forth Method) Other Method:

Compounds having a variety of functional groups may also be produced according to methods obvious to those skilled in the art or known methods, or according to their modifications. For example, starting from a compound of formula (I) and applying thereto the following reaction for converting the substituent $R^1$ or $R^2$, a part of compounds (I) may be produced.

(1) Compounds (Ie) where $R^1$ has -O-$R^0$ in compounds (I) can be produced by alkylating the compound of the invention where $R^1$ has -OH. For the reaction, for example, referred to are the methods described in Courses in Experimental Chemistry (4th Ed.), Vol. 20 by the Chemical Society of Japan (1992, by Maruzen).
(2) Compounds (If) where $R^2$ has hydroxyl group in compounds (I) can be produced by reducing the compound of the invention where $R^2$ has carbonyl group. For the reaction, for example, referred to are the methods described in Courses in Experimental Chemistry (4th Ed.), Vol. 20 by the Chemical Society of Japan (1992, by Maruzen).
(3) Compounds (Ig) where $R^1$ has phenyl group in compounds (I) can be produced through aryl-coupling reaction of the compound of the invention where $R^1$ has halogen. For the reaction, for example, referred to are the methods described in Chem. Rev., 1995, 95, 2457.

(Production of Starting Compounds)

The starting compounds used in the above-mentioned production methods may be produced, for example, according to the following reaction routes.

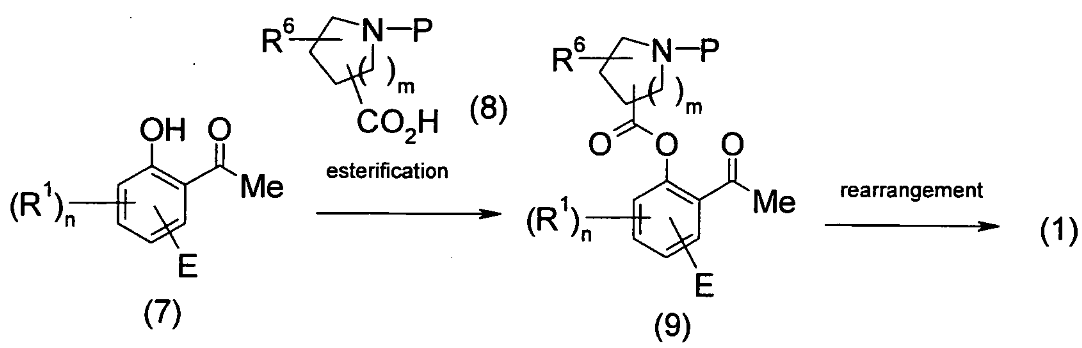

(7) (8) (9) (1)

Compound (1) can be produced by condensation through esterification of a 2-hydroxyacetophenone derivative (7) with a carboxylic acid (8) and then processing the resulting addition product (9) with a base for rearrangement.
For the esterification, employable is a method of condensing a compound (7) with a carboxylic acid (8) through dehydration in the presence of a condensing agent. For the condensing agent and the reaction condition for the case, for example, referred to are the methods described in Courses in Experimental Chemistry (4th Ed.), Vol. 22 by the Chemical Society of Japan (1992, by Maruzen). Preferably, they are reacted with from 1 to 5 equivalents of phosphorus oxychloride in a solvent of pyridine at room temperature to a refluxing temperature.
The rearrangement may be attained by treating the product with from 1 to 5 equivalents of a base such as sodium hydride or potassium tert-butoxide in a solvent of aromatic hydrocarbons or ethers, preferably at room temperature to a refluxing temperature.
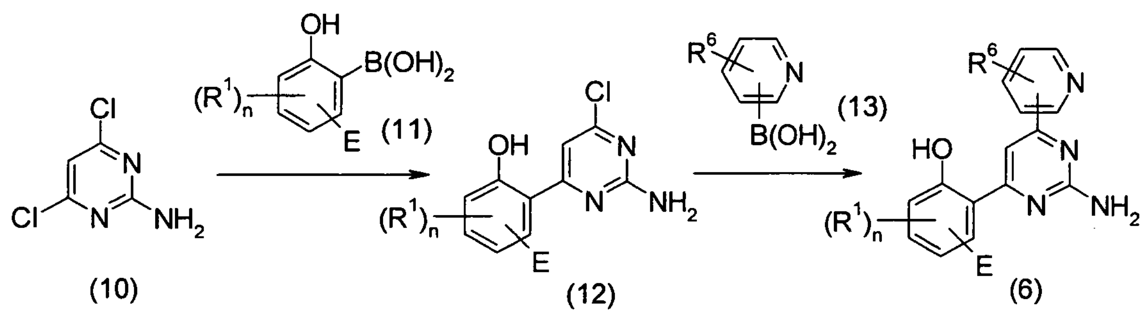

(11) (13)

(10) (12) (6)

Compound (6) can be produced through aryl-coupling reaction of a 2-amino-4,6-dichloropyrimidine (10) and a 2-hydroxyphenylboronic acid derivative (11) to give a compound (12) followed by aryl-coupling reaction of the compound (12) with a pyridylboronic acid derivative (13).
The aryl-coupling reaction may be attained in the presence of a metal catalyst, for which a palladium catalyst is preferred. For the catalyst and the reaction condition for the coupling reaction, for example, referred to are the methods described in Chem. Rev., 1995, 95, 2457.

Compound (I) thus produced can be isolated and purified as its free form, or as its salt after ordinary salt-forming treatment. The isolation and purification can be attained through ordinary chemical treatment of, for example, extraction, concentration, evaporation, crystallization, filtration, recrystallization or various chromatography.
Various isomers can be isolated in any ordinary method based on the difference in physicochemical properties between the isomers. For example, optical isomers may be separated and purified according to a method of leading a racemic compound into a diastereomer salt thereof with an optically-active organic acid (e.g., tartaric acid) and then processing the salt for fractionating crystallization; or a method of column chromatography packed with chiral stationary phase. Optically-active compounds may be produced, using a suitable optically-active compound as the starting compound. Diastereo mixtures may be separated through fractionating crystallization or chromatography.

(Test Methods)

The effect of the compound (I) serving as the active ingredient in the invention was confirmed by the following pharmacological tests.

(1) Evaluation of Rat IKK2 Enzyme Inhibition:

(i) Enzyme Preparation:

ORF of rat IKK2 (GenBank AF115282) was cloned from a rat pancreas cDNA library, and with a FLAG-tag attached thereto, it was expressed in an Sf9 cell line. The cells were dissolved in a cytolytic solution (50 mM Tris-HCl pH 7.5, 0.15M NaCl, 1% NP-40, 10% glycerol, 1 mM EDTA, 1 mM EGTA pH 7.5, 1 mM $Na_3VO_4$, 5 mM p-nitrophenylphosphate, 10 mM β-glycerophosphate, 1 mM DTT, 1 mM PMSF, 10 μg/ml leupeptin, 10 μg/ml aprotinin (by Sigma)) to prepare a large amount of an cell extract, which was then purified on an anti-FLAG M2 antibody (by Sigma). This experimental operation was carried out in accordance with gene manipulation experiment manuals such as known methods (Sambrook, J. et al, Molecular Cloning-A Laboratory Manual, Cold Spring Harbor laboratory, NY, 1989) or the instructions attached to the reagents. The purified rat IKK2 was stored in an enzyme preservative (20 mM Tris-HCl pH 7.5, 10% glycerol, 12.5 mM β-glycerophosphate, 0.5 mM EDTA, 0.5 mM EGTA, 0.05% Brij35, 1 mM DTT, 1 mM PMSF (by Sigma)) at -80°C.

(ii) Enzyme Assay:

The purified rat IKK2, 1 x enzymatic reaction buffer (20 mM Tris-HCl pH 7.5, 12.5 mM β-glycerophosphate, 20 mM $MgCl_2$, 0.1 mM DTT), 0.01% BSA (by Sigma), 0.5 μM ATP, 0.2 μM biotinated substrate peptide (18th to 49th amino acid residues of rat I-kappa B alpha (GenBank Q63746)) and a test compound dissolved in DMSO were added to a 384-well plate (catalogue No. 3677, by Corning) to be 10 μl in total therein, and left at room temperature for 90 minutes. Next, 10 μl of a reaction stopper (100 mM Hepse pH 8, 0.01% BSA, 0.8 M KF, 50 mM EDTA pH 8, 1% Triton X-100, europium cryptate-labeled anti-phosphorylation I-kappa B alpha-antibody (by Santa Cru), streptoavidin-labeled XL665 (by Nihon Schering)) was added

thereto, kept warmed for 30 minutes, and then assayed with DISCOVERY (by Perkin-Elmer). The rat IKK2 enzyme activity-inhibitory effect of the tested compound was obtained according to the following formula. Every dose was tested independently three times.

Inhibition (%) by Test Compound

= ((mean value with rat IKK2 but not with test compound) - (mean value with both test compound and rat IKK2))/((mean value with rat IKK2 but not with test compound) - (mean value not with rat IKK2)) × 100.

From the inhibition (%) at each dose, the 50 % inhibition ($IC_{50}$) of the test compound was calculated according to probit analysis. For example, the compound of Example 140 had $IC_{50}$ of 2.9 nM. The compounds of Examples 1, 5, 10, 12, 13, 14, 17, 19, 21, 22, 23, 26, 28, 37, 40, 44, 46, 47, 48, 65, 78, 81, 85, 89, 90, 92, 99, 100, 101, 103, 104, 106, 108, 126, 136, 137, 141, 142, 143, 145, 146 and 147 had $IC_{50}$ of not more than 0.5 μM.
On the other hand, in this experiment, a compound described in Patent Reference 6 (test compound 1) did not exhibit the inhibitory effect even at a dose of 10 μM.
From the results, it is confirmed that the compounds of formula (I) have a strong IKK2-inhibitory effect.

(2) Mouse LPS-Induced TNF-α Production Model:

6 weeks old Balb/c female mice were placed into two groups, a control group and a test compound adminstration group. A solution containing 10 μg/mouse of LPS in 0.9 % physiological salt was administered by intraperitoneal injection into the control mice. 60 minutes before the LPS administration, a compound of the invention was orally administered to the mice of the test compound administration group. Under anesthesia with diethyl ether, blood was collected from the posterior venous cavity of the mice of both the test compound administration group and the control group at 90 minutes after the LPS administration, and treated with heparin, and the plasma was separated from it through centrifugation at 10000 rpm at 4˚C for 10 minutes, and then diluted two-fold with PBS (pH 7.4).
The TNF-α concentration in the sample was determined with an ELISA kit (by Pharmingen, San Diego, CA). The inhibition by the mice of the test compound administration group relative to the mean inhibition by those of the control group was calculated as a mean value thereof.
The inhibition in oral administration of 30 mg/kg of compound (I) is shown below.
Example Number (inhibition): 22 (73%), 65 (75%), 79 (64%), 81 (87%), 85 (85%), 89 (87%), 103 (70%), 104 (86%), 106 (82%), 140 (86%), 141 (86%), 142 (90%).
The results confirm the strong TNF production-inhibitory effect of the compounds (I).
As so mentioned hereinabove, IKK2 is an enzyme that controls the transcription and translation of many cytokines via NF-κB, and the IKK2 inhibition results in inhibiting the production of various cytokines. This experiment is to evaluate the cytokine production-inhibitory effect of compounds (I), with reference to TNF-α, a type of cytokine that is important for the symptom of inflammation, as an index thereof. In this experiments, the compounds (I) exhibited a strong TNF production-inhibitory effect. Combined with the results of the IKK2 enzyme-inhibitory effect of the compounds (I) as in the above, the results in this experiment suggest the possibility that the compounds (I) have the ability to inhibit the transcription and translation of a variety of cytokines based on their ability to inhibit IKK2. In fact, the following in-vivo experiments confirmed a good antiinflammatory effect of the compounds (1).

(3) Rat Carrageenan Paw Edema Model:

After their body weight was measured, Sprague-Dawley male rats (6-10 weeks age, male, by Nippon SLC) were divided into groups of 5 rats each in such a manner that the mean body weight of each group could be on the same level. Next, a test compound was orally administered to the rats (but 10 mL/kg of solvent alone was administered to those of the control group). 30 minutes after the test compound administration thereto, 100 μL of a 1 % carrageenan (by Sigma Aldrich Japan) solution was subcutaneously injected to the sub-plantar of the right foot of each rat to induce inflammation. 3 hours after the inflammation induction, the rats were killed by deep anesthetization with ether, and their tissue below the right and left ankles was cut and collected and its weight was measured.
The test result was obtained as follows: In every rat, the weight of the left foot with no carrageenan injection thereto was subtracted from the weight of the right foot with carrageenan injection thereto, 3 hours after the carrageenan injection, thereby calculating the weight difference (g) therebetween. The edema inhibition in the test compound-administered

rats was calculated relative to the mean value of the rats of the control group. For multi-group comparison between the control group and the test compound administration group, employed was a Dunnett multiple comparison test, in which the P value less than 0.05 was considered as statistical significance. All the above statistical analyses were carried out, using SAS.
In this experiment, the compounds (I) exhibited an excellent antiinflammatory effect. For example, the compound of Example 21 exhibited 30 % inhibitory activity in 30 mg/kg oral administration. The compound of Example 23 exhibited 50 % inhibitory activity.
On the other hand, a compound described in Patent Reference 6 (test compound 1) did not exhibit a significant inhibitory effect at a dose of 50 mg/kg thereof in this experiment. Methotrexate, an ordinary antirheumatic drug, also did not exhibit an effect in this experiment.
The results confirm the excellent acute inflammation-inhibitory effect of the IKK2 inhibitor of the invention.

(4) Collagen-Induced Rheumatoid Arthritis Model:

The effect to rat collagen-induced rheumatoid arthritis was evaluated according to a partial modification of the method described in The Japanese Journal of Pharmacology, 1997 Aug; 74(4): 313-22.
10 mL of a 1 mg/mL solution of bovine-derived type 2 collagen (by Cosmobio) diluted three-fold with acetic acid was mixed with adjuvant incomplete Freund (by Difco) in 1/1, and emulsified to give an emulsion for sensitization. The tail root of each rat in a control group and in a test compound administration group was disinfected with ethanol, and collagen-sensitized through intradermal injection of 0.4 mL of the emulsion containing 200 μg of collagen thereinto to thereby induce rheumatoid arthritis. The rats in the non-sensitized group were not treated for collagen sensitization. To the rats of the control group, 10 mL/kg of 0.5 % methyl cellulose was orally administered. To the rats of the test compound administration group, a compound of the invention was orally administered. One week after the sensitization, the administration continued for 14 days. The degree of inflammation was judged by measuring the rheumatoid arthritis scores of the four limbs of each rat (scoring). Briefly, the symptom of each articulation was visually grouped into the following four ranks (0: normal, 1: flare or light swelling, 2: middle swelling, 3: heavy swelling or joint ankylosis); and the total of the numerical data of the four limbs is referred to as the rheumatoid arthritis score. The scoring was carried out every other day after the development of rheumatoid arthritis. The cumulative rheumatoid arthritis score was calculated by totaling the rheumatoid arthritis scores on every scoring date of every individual. Relative to the mean cumulative rheumatoid arthritis score of rats of the control group, the inhibition by the those of the test compound administration group was calculated, and the mean value of the data was shown.
In this experiment, the compounds (I) exhibited an excellent antiinflammatory effect. For example, the compounds of Examples 37, 85 and 92 exhibited nearly 100 % inhibition in oral administration at a dose of 30 mg/kg.
The effect to mouse collagen-induced rheumatoid arthritis was evaluated according to the method described in The Japanese Journal of Pharmacology, 2002 Apr; 88(3): 332-340.
The results confirm the excellent therapeutical effect to chronic inflammation of the IKK2 inhibitor of the invention.
The above-mentioned test results confirm that the compounds of the invention have a strong IKK2-inhibitory activity, and are therefore effective against production of cytokines and further against both acute inflammations and chronic inflammations.
The pharmaceutical composition serving as an IKK2 inhibitor of the invention may be prepared, using carriers, vehicles and other additives usable in ordinary pharmaceutical preparations.
The administration of the composition may be in any route of oral administration as tablets, pills, capsules, granules, powders or liquids; or parenteral administration as intravenous or intramuscular injections or as external preparations such as suppositories, percutaneous preparations, nasal preparations or inhalants. Their dose may be suitably determined, depending on the condition, the age and the sex of the patients to which they are administered, but is, in general, from 0.001 to 100 mg/kg adult/day or so for oral administration. This may be administered to the patients all at a time, or may be divided into a few portions for administration in 2 to 4 times a day. For intravenous administration depending on the condition of the patients, in general, the dose may be from 0.0001 to 10 mg/kg adult/day or so. This may be administered to the patients all at a time, or may be divided into a few portions for administration in plural times a day. For inhalation, in general, the dose may be from 0.0001 to 1 mg/kg adult/day or so. This may be inhaled by the patients all at a time, or may be divided into a few portions for inhalation in plural times a day.
As the solid composition for oral administration of the invention, employed are tablets, powders, granules, etc. The solid composition of those types comprises one or more active substances along with at least one inert vehicle, such as lactose, mannitol, glucose, hydroxypropyl cellulose, microcrystalline cellulose, starch, polyvinyl pyrrolidone, magnesium metasilicate aluminate. In an ordinary manner, the composition may contain any inert additives, for example, a lubricant such as magnesium stearate, a disintegrator such as sodium carboxymethyl starch, and a dissolution promoter. If desired, the tablets and pills may be coated with sugar or with a gastric or enteric coating agent.
The liquid composition for oral administration includes pharmaceutically-acceptable emulsions, solutions, suspensions, syrups, elixirs, which contain an ordinary inert solvent such as pure water or ethanol. In addition to the inert solvent, those compositions may further contain pharmaceutical aids such as solubilizers, wetting promoters, suspension promoters, and also sweeteners, flavorings, aromas and preservatives.

Injection for parenteral administration includes, for example, germ-free, aqueous or non-aqueous solutions, suspensions and emulsions. The aqueous solvent includes, for example, distilled water and physiological saline for injections. The non-aqueous solvent includes, for example, propylene glycol, polyethylene glycol, vegetable oils such as olive oil, alcohols such as ethanol, Polysolvate 80 (trade name). Those compositions may further contain additives such as isotonizers, preservatives, wetting promoters, emulsifiers, dispersants, stabilizers, dissolution promoters. These are sterilized by filtering them through bacteria-trapping filters, or by adding germicides thereto, or by exposing them to radiations. Germ-free, solid compositions may be produced previously, and they may be dissolved in germ-free water or in germ-free solvents for injection, before using them.

The transmucomembranous preparations such as inhalants and nasal preparations may be solid, liquid or gel, and they may be produced in any known methods. For example, a vehicle such as lactose or starch, and further a pH-controlling agent, a preservative, a surfactant, a lubricant, a stabilizer and a viscosity-increasing agent may be suitably added to them. For their administration, a suitable device for inhalation or blowing introduction may be used. For example, a known device or spray such as a device for metered dose inhalation may be used, with which the compound may be administered either singly as it is or as a powder of a formulated mixture containing it, or as a solution or suspension of the compound as combined with a pharmaceutically-acceptable carrier. The dry powder inhaler may be for single administration or for multiple administration, for which a dry powder or a powder-containing capsule may be used. As the case may be, a pressure aerosol spray or the like may also be used, that contains a suitable propellant, for example, a favorable vapor such as chlorofluoroalkane, hydrofluoroalkane or carbon dioxide.

The preparation for external application include ointments, plasters, creams, jellies, poultices, sprays, lotions, eye drops, eye ointments. They may contain any ordinary ointment bases, lotion bases, aqueous or non-aqueous liquids, suspensions or emulsions. For example, ointment or lotion bases include polyethylene glycol, carboxyvinyl polymer, white petrolatum, bleached bees wax, polyoxyethylene-hardened castor oil, glycerin monostearate, stearyl alcohol, cetyl alcohol, lauromacrogol, sorbitan sesquioleate.

EXAMPLES

Methods for producing the compounds (I) of the invention are described in detail with reference to the following Examples. The compounds of the invention should not be limited to the compounds described in the following Examples. Methods for producing the starting compounds are shown in Reference Examples.

Reference Example 1:

(Bromomethyl)cyclopropane was added to a mixture of 1-(2,6-dihydroxy-4-methylphenyl)ethanone, potassium carbonate, potassium iodide and DMF, and reacted at 60˚C for 4 hours to obtain 1-[2-(cyclopropylmethoxy)-6-hydroxy-4-methylphenyl]ethanone.
FAB-MS$(M+H)^+$: 221.

Reference Example 2:

Titanium tetrachloride was added to 4-(methylsulfanyl)phenol in an argon atmosphere, and stirred at room temperature for 2 hours. Next, acetyl chloride was added thereto and reacted at 120˚C for 1 hours to obtain 1-[2-hydroxy-5-(methylsulfanyl)phenyl]ethanone.
ESI-MS$(M-H)^-$: 181.

Reference Example 3:

9-Borabicyclo[3.3.1]nonane (9-BBN) dimer was added to a THF solution of ethyl 7-oxoazepane-4-carboxylate, and stirred at 60˚C for 3 hours, then left cooled to room temperature, and ethanolamine was added thereto, and diluted with n-pentane. The resulting white precipitate was removed through Celite filtration, and the solvent of the filtrate was evapolated to obtain ethyl 7-azepane-4-carboxylate.
FAB-MS$(M+H)^+$: 172.

Reference Example 4:

Di-tert-butyl dicarbonate was added to a chloroform solution of ethyl 7-azepane-4-carboxylate under ice-cooling,

and stirred overnight at room temperature, and the solvent was evaporated to obtain 1-tert-butyl-4-ethyl azepane-1,4-dicarboxylate. The compound was dissolved in methanol, and hydrolyzed with potassium hydroxide (1.0 equivalent) in methanol to obtain 1-(tert-butoxycarbonyl)azepane-4-carboxylic acid.
FAB-MS$(M+H)^+$: 244.

Reference Example 5:

Under ice-cooling, phosphorus oxychloride was added to a mixed solution of 1-(tert-butoxycarbonyl)piperidine-3-carboxylic acid, 2-hydroxyacetophenone and pyridine, and then reacted at 60˚C for 30 minutes to obtain 3-(2-acetylphenyl)-1-tert-butyl piperidine-1,3-dicarboxylate.
FAB-MS$(M+H)^+$: 348.

Reference Example 6:

Under ice-cooling, sodium hydride was added to a THF solution of 3-(2-acetylphenyl)-1-tert-butyl piperidine-1,3-dicarboxylate, and then reacted at 60˚C for 30 minutes to obtain tert-butyl 3-[3-(2-hydroxyphenyl)-3-oxopropanoyl] piperidine-1-carboxylate.
FAB-MS$(M+H)^+$: 348.
In the same manner as in Reference Example 1, compounds of Reference Examples 7 to 12 as in Table 1 below were obtained; in the same manner as in Reference Example 5, compounds of Reference Examples 13 to 31 as in Table 2 below and compounds of Reference Examples 32 to 42 as in Table 3 below were obtained; in the same manner as in Reference Example 6, compounds of References 43 to 65 as in Table 4 below and compounds of Reference Examples 66 to 72 as in Table 5 below were obtained; and in a continuous two-step process to be carried out in the same manner as in Reference Example 5 and Reference Example 6, compounds of Reference Examples 73 to 75 as in Table 6 below were obtained, all from the corresponding starting materials.
The structures and the physicochemical data of the compounds of Reference Examples 7 to 75 are shown in Tables 1 to 6.

Reference Example 76:

(1) 2-Hydroxyphenyl boric acid, tetrakis(triphenylphosphine) palladium and aqueous 2 M sodium carbonate solution were added to a 1,4-dioxane solution of 2-amino-4,6-dichloropyrimidine, and stirred overnight at 100˚C. The reaction mixture was treated in an ordinary manner to obtain 2-(2-amino-6-chloropyrimidin-4-yl)phenol as a bright grayish yellow solid.
(2) Next, 6-methylpyridine-3-ylboric acid, tetrakis(triphenylphosphine) palladium and aqueous 2 M sodium carbonate solution were added to a 1,4-dioxane solution of the above compound, and stirred overnight at 95˚C. The reaction mixture was processed in an ordinary manner to obtain 2-[2-amino-6-(6-methylpyridin-3-yl)pyrimidin-4-yl]phenol as a yellow solid.

FAB-MS$(M+H)^+$: 279.

Reference Example 77:

500 mg of 10 % palladium-carbon was added to a solution of 27 g of tert-butyl 3-{3-[2-(benzyloxy)-6-hydroxyphenyl]-3-oxopropanoyl}piperidine-1-carboxylate in a mixture of 250 ml of methanol and 150 ml of ethyl acetate, and stirred in a hydrogen atmosphere for 3 hours. The reaction mixture was filtered through Celite, and the solvent was evaporated to obtain 21.5 g of 3-[3-(2,6-dihydroxyphenyl)-3-oxopropanoyl]piperidin-1-carboxylate as a gray solid.
FAB-MS$(M+H)^+$: 364.
In the same manner as in Reference Example 4, a compound of Reference Example 90 shown in Table 9 below was obtained; in the same manner as in Reference Example 5, compounds of Reference Examples 78 to 83 as in Table 7 below and a compound of Reference Example 91 as in Table 9 below were obtained; in the same manner as in Reference Example 6, compounds of Reference Examples 84 to 89 as in Table 8 below and a compound of Reference Example 92 as in Table 9 below were obtained; and in the same manner as in Reference Example 8, compounds of Reference Examples 93 and 94 as in Table 9 below were obtained, all from the corresponding starting materials.
The structures and the physicochemical data of the compounds of Reference Examples 78 to 94 are shown in Tables 7 to 9.

Example 1:

(1) Under ice-cooling, 1.73 g of sodium hydride was added to a THF (50 ml) solution of 4.12 g of guanidine hydrochloride, and stirred at room temperature for 1 hour. The reaction mixture was concentrated, and 50 ml of ethanol and 5.00 g of tert-butyl 3-[3-(2-hydroxyphenyl)-3-oxopropanoyl]piperidine-1-carboxylate were added thereto, and stirred at 70˚C for 8 hours. The reaction mixture was concentrated, and 150 ml of water was added to the resulting residue, neutralized with aqueous 1 M hydrochloric acid solution, and extracted with 150 ml of ethyl acetate. The resulting organic layer was washed with brine (100 ml), dried with anhydrous sodium sulfate, and the solvent was evaporated. The resulting residue was purified through silica gel column chromatography (hexane/ethyl acetate = 9/1 to 2/1) to obtain 3.77 g of tert-butyl 3-[2-amino-6-(2-hydroxyphenyl)pyrimidin-4-yl]piperidine-1-carboxylate as a yellow solid.
(2) Next, 60 ml of 2 M hydrogen chloride-dioxane solution was added to 3.77 g of the compound, and stirred at room temperature for 19 hours. The reaction mixture was concentrated, and the resulting solid was washed with acetonitrile to obtain 3.52 g of 2-(2-amino-6-piperidin-3-ylpyrimidin-4-yl)phenol dihydrochloride as a yellow powder.

Example 2:

422 mg of potassium carbonate and 0.05 ml of methyl iodide were added to an acetonitrile/DMF (1/1, 5 ml) mixed solution of 300 mg of 2-(2-amino-6-piperidin-3-ylpyrimidin-4-yl)phenol, and the mixture was stirred at room temperature for 1 hour. The reaction mixture was concentrated, and 50 ml of water and 50 ml of chloroform were added thereto, and the precipitated solid was filtered off. The filtrate was separated, the resulting organic layer was dried with anhydrous sodium sulfate, and the solvent was evaporated. The resulting residue was purified through silica gel column chromatography (chloroform/methanol/aqueous ammonia = 99/1/0.1 to 90/10/1), and the resulting solid was washed with a mixed solvent of acetonitrile-diethyl ether to obtain 35 mg of 2-[2-amino-6-(1-methylpiperidin-3-yl)pyrimidin-4-yl] phenol as a pale yellow powder.

Example 3:

In the same manner as in Example 1(1) but using 18.26 g of guanidine hydrochloride and 28.89 g of tert-butyl 3-{3-[2-(benzyloxy)-6-hydroxyphenyl]-3-oxopropanoyl}piperidine-1-carboxylate as the starting compounds, 11.11 g of tert-butyl 3- {2-amino-6-[2-(benzyloxy)-6-hydroxyphenyl]pyrimidin-4-yl}piperidine-1-carboxylate as a pale green powder.

Example 4:

25 ml of methanol, 25 ml of acetonitrile, 10 ml of DMF and 200 mg of 10 % palladium-carbon were added to 2.00 g of tert-butyl 3-{2-amino-6-[2-(benzyloxy)-6-hydroxyphenyl]pyrimidin-4-yl}piperidine-1-carboxylate, and stirred in a hydrogen atmosphere at room temperature for 4.5 hours. The reaction mixture was filtered through Celite, and the filtrate was concentrated. The resulting solid was washed with chloroform to obtain 1.43 g of tert-butyl 3-[2-amino-6-(2,6-dihydroxyphenyl)pyrimidin-4-yl]piperidine-1-carboxylate as a pale green powder.

Example 5:

(1) 151 ml of ethyl iodide was added to a solution of 310 mg of tert-butyl 3-[2-amino-6-(2,6-dihydroxyphenyl)pyrimidin-4-yl]piperidine-1-carboxylate, 556 mg of potassium carbonate and 6 ml of DMF, and stirred at 50˚C for 16 hours. 50 ml of ethyl acetate was added to the reaction mixture, and washed with 50 ml of aqueous saturated ammonium chloride solution. The resulting organic layer was dried with anhydrous magnesium sulfate, and the solvent was evaporated. The resulting solid was washed with ethyl acetate to obtain 166 mg of tert-butyl 3-[2-amino-6-(2-ethoxy-6-hydroxyphenyl)pyrimidin-4-yl]piperidine-1-carboxylate as a yellow powder.
(2) Next, 6 ml of 2 M hydrogen chloride-ethyl acetate solution was added to 156 mg of the above compound, and stirred at room temperature for 12 hours. The reaction mixture was filtered, and the resulting solid was washed with ethyl acetate to obtain 102 mg of 2-(2-amino-6-piperidin-3-ylpyrimidin-4-yl)-3-ethoxyphenol dihydrochloride as a yellow powder.

Example 6:

1.60 g of sodium acetoxyborohydride was, as divided into 3 portions, added separately in three times to 1,2-dichloroethane/dimethylformamide (5/3, 8 ml) mixed solution of 300 mg of 2-(2-amino-6-piperidin-4-ylpyrimidin-4-yl)-4-methylphenol dihydrochloride and 128 mg of 1H-indole-3-carbaldehyde, and stirred at room temperature for 29.5 hours. 50 ml of chloroform was added to the reaction mixture, and washed with 40 ml of aqueous saturated sodium bicarbonate solution. The resulting organic layer was dried with anhydrous sodium sulfate, and the solvent was evaporated. The resulting residue was purified through silica gel column chromatography (chloroform/methanol solution/aqueous ammonia = 99/1/0.1 to 96/4/0.4). 0.20 ml of 4 M hydrogen chloride-ethyl acetate solution was added to ethyl acetate (5 ml) solution of 152 mg of the obtained pale yellow amorphous solid. The reaction mixture was concentrated, and the resulting solid was washed with acetonitrile to obtain 125 mg of 2-{2-amino-6-[1-(1H-indol-3-ylmethyl)piperidin-4-yl]pyrimidin-4-yl}phenol dihydrochloride as a yellow powder.

Example 7:

0.2 ml of triethylamine was added to DMF (8 ml) solution of 246 mg of 2-(2-amino-6-pyrrolidin-3-ylpyrimidin-4-yl)-4-methylphenol dihydrochloride, 149 mg of 5-phenyl-1,3-oxazole-4-carboxylic acid, 107 mg of 1-hydroxybenzotriazole (HOBt) and 151 mg of 1-(3-dimethylaminopropyl)-3-ethylcarbodiimide hydrochloride (WSC·HCl), and stirred overnight at room temperature. The reaction solution was concentrated, and purified through silica gel column chromatography (chloroform/methanol = 98/2). 3 ml of 4 M hydrochloric acid-dioxane solution was added to a methanol solution of the resulting product, and stirred for 5 minutes. Then, the solvent was evaporated, and the resulting residue was recrystallized from methanol-diethyl ether to obtain 246 mg of 2-(2-amino-6-{1-[(5-phenyl-1,3-oxazol-4-yl)carbonyl]pyrrolidin-3-yl}pyrimidin-4-yl)-4-methylphenol hydrochloride as a yellow powder.

Example 8:

A mixture of 100 mg of ethyl {3-[2-amino-6-(5-ethyl-2-hydroxyphenyl)pyrimidin-4-yl]piperidin-1-yl}acetate and 1 ml of aqueous 6 M hydrochloric acid solution was stirred at 100˚C for 9 hours. The reaction mixture was cooled, then the solvent was evaporated, and 5 ml of toluene was added to the resulting residue. The solvent was again evaporated. The resulting residue was washed with ethyl acetate to obtain 111 mg of {3-[2-amino-6-(5-ethyl-2-hydroxyphenyl)pyrimidin-4-yl]piperidin-1-yl}acetic acid dihydrochloride as a yellow powder.

Example 9:

Under ice-cooling, 440 mg of sodium borohydride was, as divided into 8 portions, added separately in 8 times to a methanol (5 ml) solution of 195 mg of 2-{4-[2-amino-6-(2-hydroxy-5 -methylphenyl)pyrimidin-4-yl]piperidin-1-yl}-1-phenylethanone. After the reaction mixture was stirred at room temperature for 26 hours, 50 ml of ethyl acetate was added. The reaction mixture was washed with 50 ml of water and 30 ml of brine in that order. The resulting organic layer was dried with anhydrous sodium sulfate, and then the solvent was evaporated. The resulting residue was purified through silica gel column chromatography (chloroform/methanol = 99/1 to 96/4), and then, Under ice-cooling, 3 ml of ethyl acetate and 0.07 ml of 4 M hydrogen chloride-ethyl acetate solution were added thereto. The reaction mixture was concentrated, and the resulting solid was washed with a mixed solvent acetonitrile/methanol (10/1) to obtain 56 mg of 2-{2-amino-6-[1-(2-hydroxy-2-phenylethyl)piperidin-4-yl]pyrimidin-4-yl}-4-methylphenol dihydrochloride as a yellow powder.

Example 10:

In the same manner as in Example 1(2), 73 mg of 2-(2-amino-6-piperidin-3-ylpyrimidin-4-yl)benzene-1,3-diol dihydrochloride was obtained from 169 mg of tert-butyl 3-[2-amino-6-(2,6-dihydroxyphenyl)pyrimidin-4-yl]piperidine-1-carboxylate as a pale yellow solid.

Example 11:

(1) In the same manner as in Example 7, 459 mg of tert-butyl [(1R)-2-{3-[2-amino-6-(2-hydroxy-5-methylphenyl) pyrimidin-4-yl]pyrimidin-1-yl}-1-(4-fluorophenyl)-2-oxoethyl]carbamate was obtained from 246 mg of 2-(2-amino-6-pyrrolidin-3-ylpyrimidin-4-yl)-4-methylphenol and 212 mg of (2R)-[(tert-butoxycarbonyl)amino](4-fluorophenyl)acetic

acid as a yellow oil.

(2) Next, in the same manner as in Example 1(2), 247 mg of 2-(2-amino-6-{1-[(2R)-2-amino-2-(4-fluorophenyl)acetyl] pyrrolidin-3-yl}pyrimidin-4-yl)-4-methylphenol dihydrochloride was obtained from the above compound as a yellow powder.

In the same manner as in the above-mentioned Examples, compounds of Examples 12 to 64 were prepared. Their structural formulae and physical properties are shown in Tables 10 to 15 below.

Example 65:

(1) Under ice-cooling, 357 mg of di-tert-butyl azodicarboxylate was added to a THF (6 mL) solution of 300 mg of tert-butyl 3-[2-amino-6-(2,6-dihydroxyphenyl)pyrimidin-4-yl]piperidine-1-carboxylate, 133 mg of (2-methylcyclopropyl)methanol and 407 mg of triphenyl phosphine, and stirred for 2 hours. The solvent was evaporated, and the resulting residue was purified through silica gel column chromatography (ethyl acetate/hexane solution = 2/1). The resulting pale yellow solid was recrystallized from a mixed solution of ethyl acetate-hexane to obtain 178 mg of tert-butyl 3-(2-amino-6-{2-hydroxy-6-[(2-methylcyclopropyl)methoxy]phenyl}pyrimidin-4-yl)piperidine-1-carboxylate as a pale yellow solid.

(2) Next, 178 mg of the above compound was treated in the same manner as in Example 1(2) to obtain 125 mg of 2-(2-amino-6-piperidin-3-ylpyrimidin-4-yl)-3-[(2-methylcyclopropyl)methoxy]phenol dihydrochloride as a yellow powder.

Example 66:

(1) 104 mg of α-bromo-p-xylene was added to a solution of 200 mg of tert-butyl 3-[2-amino-6-(2,6-dihydroxyphenyl) pyrimidin-4-yl]piperidine-1-carboxylate, 108 mg of potassium carbonate and 5 ml of DMF, and stirred at room temperature for 20 hours. Water was added to the solution, and the precipitated crystal was taken out through filtration, and recrystallized from ethyl acetate to obtain 136 mg of tert-butyl 3-(2-amino-6-{2-hydroxy-6-[(4-methylbenzyl)oxy] phenyl}pyrimidin-4-yl)piperidine-1-carboxylate as a white solid.

(2) Next, 316 mg of trifluoroacetic acid (TFA) was added to dichloroethane (3 ml) solution of 136 mg of the above compound, and stirred at room temperature for 15 hours, and then the solvent was evaporated. The residue was made alkaline with aqueous saturated sodium bicarbonate solution added thereto, and then stirred for 30 minutes. The precipitated solid was taken out through filtration, dissolved in THF, and the insoluble matter was separated through filtration. The solvent was evaporated to obtain 116 mg of 2-(2-amino-6-piperidin-3-ylpyrimidin-4-yl)-3-[(4-methylbenzyl)oxy]phenol. The obtained compound was dissolved in a mixed solution of THF and methanol, and 27 mg of fumaric acid was added thereto and stirred, and thereafter the solvent was evaporated. The resulting residue was washed with ethanol to obtain 64 mg of 2-(2-amino-6-piperidin-3-ylpyrimidin-4-yl)-3-[(4-methylbenzyl)oxy]phenol monofumarate as a pale yellow solid.

Example 67:

(1) 0.67 g of 40 % toluene solution of diethyl azodicarboxylate (DEAD) was added to THF (8 ml) solution of 300 mg of tert-butyl 3-[2-amino-6-(2,6-dihydroxyphenyl)pyrimidin-4-yl]piperidine-1-carboxylate, 123 mg of (2,2-dimethyl-1,3-dioxolan-4-yl)methanol and 407 mg of triphenyl phosphine, and stirred overnight at room temperature. The reaction mixture was concentrated under reduced pressure, and the resulting residue was purified through silica gel column chromatography (hexane/ethyl acetate = 3/1 to 1/1) to obtain 450 mg of tert-butyl 3-{2-amino-6-[2-(2,3-dihydroxypropoxy)-6-hydroxyphenyl]pyrimidin-4-yl}piperidine-1-carboxylate as a yellow oil.

(2) Next, 0.60 ml of TFA was added to a solution of 450 mg of the above compound in 6 ml of dichloromethane, and stirred overnight at room temperature. The reaction mixture was diluted with ethyl acetate, and extracted with aqueous 1 M hydrochloric acid. The resulting aqueous layer was controlled to have a pH of 10 with aqueous 10 M sodium hydroxide solution, and then extracted with chloroform/2-propanol (5/1) mixed solution. The resulting organic layer was dried with anhydrous sodium sulfate, and the solvent was evaporated. The obtained solid was dissolved in 5 ml of methanol, and 40 mg of fumaric acid was added thereto and stirred, and then concentrated under reduced

pressure. The resulting residue was recrystallized from ethyl acetate-acetone to obtain 100 mg of 3-[2-(2-amino-6-piperidin-3-ylpyrimidin)-3-hydroxyphenoxy]propane-1,2-diol as a pale yellow powder.

Example 68:

(1) Chloroform (5 ml) solution of 183 mg of m-chloroperbenzoic acid (mCPBA) was added to chloroform (20 ml) solution of 335 mg of tert-butyl 3-{2-amino-6-[2-hydroxy-5-(methylsulfanyl)phenyl]pyrimidin-4-yl}piperidine-1-carboxylate, and stirred at room temperature for 5 hours. Aqueous saturated sodium bicarbonate solution was added to the reaction mixture, and extracted with chloroform. The resulting organic layer was washed with water, then dried with anhydrous sodium sulfate, then the solvent was evaporated. The resulting residue was purified through silica gel column chromatography (ethyl acetate) to obtain 345 mg of tert-butyl 3-{2-amino-6-[2-hydroxy-5-(methylsulfmyl)phenyl]pyrimidin-4-yl}piperidine-1-carboxylate as a yellow solid.

(2) Next, 1.7 ml of 4 M hydrogen chloride-dioxane solution was added to dioxane (3 ml) solution of 345 mg of the above compound, and stirred for 20 minutes. The crystal was taken out through filtration to obtain 178 mg of 2-(2-amino-6-piperidin-3-ylpyrimidin-4-yl)-4-(methylsulfmyl)phenol dihydrochloride as a yellow powder.

Example 69:

300 mg of tert-butyl 3-{2-amino-6-[2-hydroxy-5-(methylsulfanyl)phenyl]pyrimidin-4-yl}piperidine-1-carboxylate and 461 mg of mCPBA in chloroform were reacted at room temperature for 2 hours, and then treated in the same manner as in Example 69 to obtain 154 mg of 2-(2-amino-6-piperidin-3-ylpyrimidin-4-yl)-4-(methylsulfonyl)phenol dihydrochloride as a yellow powder.

Example 70:

(1) Under ice-cooling, 1.09 g of 60 % sodium hydride and 9.7 g of N-phenyltrifluoromethanesulfonimide were added to DMF (50 ml) solution of 500 mg of tert-butyl 3-[2-amino-6-(2,6-dihydroxyphenyl)pyrimidin-4-yl]piperidine-1-carboxylate, and stirred for 30 minutes. Aqueous saturated ammonium chloride solution was added to the reaction mixture, and extracted with ethyl acetate. The organic layer was washed with brine, dried with anhydrous magnesium sulfate, and the solvent was evaporated. The resulting residue was purified through silica gel column chromatography (hexane/ethyl acetate solution = 5/1 to 4/1) to obtain 7.51 g of tert-butyl 3-[2-amino-6-(2,6-bis{[(trifluoromethyl)sulfonyl]oxy}phenyl)pyrimidin-4-yl]piperidine-1-carboxylate as a colorless amorphous solid.
FAB-MS: 651 $[(M+H)^+]$

(2) Next, 4.03 g of vinyltributyltin, 1.47 g of lithium chloride, 664 mg of tetrakistriphenylphosphine palladium and 127 mg of 2,6-di-tert-butyl-p-cresol were added to dioxane (100 ml) solution of 7.51 g of the above compound, and stirred in an argon atmosphere at 100˚C for 5 hours. 15 ml of aqueous 10 % potassium fluoride solution was added to the reaction mixture, stirred for 15 minutes, then filtered through Celite, and the solvent was evaporated. 45 ml of aqueous 1 N sodium hydroxide solution was added to ethanol (60 ml) solution of the resulting residue, then stirred at 80˚C for 20 hours, and the solvent was evaporated. Aqueous saturated ammonium chloride solution was added to the resulting residue, and extracted with ethyl acetate. The resulting organic layer was washed with brine, dried with anhydrous magnesium sulfate, and the solvent was evaporated. The residue was purified through silica gel column chromatography (hexane/ethyl acetate solution = 3/1) to obtain 1.73 g of tert-butyl 3-[2-amino-6-(2-hydroxy-6-vinylphenyl)pyrimidin-4-yl]piperidine-1-carboxylate as a pale yellow solid.
FAB-MS: 397 $[(M+H)^+]$

(3) Next, 484 mg of TFA was added to dichloroethane (5 ml) solution of 160 mg of the above compound, and stirred at room temperature for 20 hours. Then, the solvent was evaporated, and the residue was made alkaline with aqueous 1 M sodium hydroxide solution added thereto. The solution was extracted with ethyl acetate, the resulting organic layer was washed with brine, and dried with anhydrous magnesium sulfate, and the solvent was evaporated. The residue was dissolved in a mixed solvent of ethyl acetate, THF and water, and a small amount of 4 M hydrogen chloride-ethyl acetate solution was added thereto and stirred for 30 minutes, then the solvent was evaporated. The resulting residue was washed with acetone to obtain 105 mg of 2-(2-amino-6-piperidin-3-ylpyrimidin-4-yl)-3-vinylphenol 1.5-hydrochloride as a pale yellow solid.

Example 71:

(1) 820 mg of benzylamine was added to dioxane (5 ml) solution of 1 g of tert-butyl 3-[2-amino-6-(2,6-bis{[(trifluoromethyl)sulfonyl]oxy}phenyl)pyrimidin-4-yl]piperidine-1-carboxylate, and stirred at 100˚C for 6 days. Ethyl acetate was added to the reaction mixture, washed with brine, dried with anhydrous magnesium sulfate, and the solvent was evaporated. The resulting residue was purified through silica gel column chromatography (hexane/ethyl acetate = 6/1 to 3/1) to obtain 553 mg of tert-butyl 3-[2-amino-6-(2-(benzylamino)-6-{[(trifluoromethyl)sulfonyl]oxy}phenyl) pyrimidin-4-yl]piperidine-1-carboxylate as a yellow amorphous solid. Next, 100 mg of aqueous 8 M sodium hydroxide solution was added to ethanol (1 ml) solution of 200 mg of the above compound, and stirred for 18 hours. Aqueous saturated ammonium chloride solution was added to the reaction mixture, and extracted with ethyl acetate. The resulting organic layer was washed with brine, dried with anhydrous magnesium sulfate, and the solvent was evaporated. The resulting residue was purified through silica gel column chromatography (hexane/ethyl acetate solution = 20/1 to 6/1) to obtain 80 mg of tert-butyl 3-{2-amino-6-[2-(benzylamino)-6-hydroxyphenyl]pyrimidin-4-yl}piperidine-1-carboxylate as a yellow oil.
(2) Next, 4 ml of 4 M hydrogen chloride-ethyl acetate solution was added to ethyl acetate (4 ml) solution of 80 mg of the above compound, and stirred for 3 hours. The precipitated solid was taken out through filtration and washed with acetonitrile to obtain 71 mg of 2-(2-amino-6-piperidin-3-ylpyrimidin-4-yl)-3-(benzylamino)phenol trihydrochloride as a white solid.

Example 72:

(1) Under ice-cooling, 1.38 g of 4-methylmorpholine 4-oxide and 2.5 ml of 2.5 % osmium tetroxide tert-butanol solution were added to a solution of 1.56 g of tert-butyl 3-[2-amino-6-(2-hydroxy-6-vinylphenyl)pyrimidin-4-yl]piperidine-1-carboxylate in 40 ml of THF and 30 ml of water, and stirred at room temperature for 12 hours. 30 ml of aqueous 10 % sodium sulfite solution was added to the reaction mixture, and stirred, and then extracted with ethyl acetate. The resulting organic layer was washed with brine, dried with anhydrous magnesium sulfate, and the solvent was evaporated. The resulting residue was purified through silica gel column chromatography (chloroform/methanol = 100/0 to 20/1) to obtain 1.27 g of tert-butyl 3-{2-amino-6-[2-(1,2-dihydroxyethyl)-6-hydroxyphenyl]pyrimidin-4-yl} piperidine-1-carboxylate as a pale yellow amorphous solid.
(2) Next, 2 ml of 4 M hydrogen chloride-ethyl acetate solution was added to ethyl acetate (2 ml) solution of 52 mg of the above compound, and stirred for 4 hours. The precipitated solid was taken out through filtration, and washed with ethyl acetate to obtain 38 mg of 1-[2-(2-amino-6-piperidin-3-ylpyrimidin-4-yl)-3-hydroxyphenyl]ethane-1,2-diol dihydrochloride as a white powder.

Example 73:

(1) 50 mg of 10 % palladium-carbon was added to ethyl acetate (10 ml) solution of 130 mg of tert-butyl 3-[2-amino-6-(2-hydroxy-6-vinylphenyl)pyrimidin-4-yl]piperidine-1-carboxylate, and stirred in a hydrogen atmosphere for 3 hours. The reaction mixture was filtered through Celite, and the solvent was evaporated. The resulting residue was purified through silica gel column chromatography (hexane/ethyl acetate = 2/1 to 1/1) to obtain 83 mg of tert-butyl 3-[2-amino-6-(2-ethyl-6-hydroxyphenyl)pyrimidin-4-yl]piperidine-1-carboxylate as a colorless oil.
(2) Next, 4 ml of 4 M hydrogen chloride-ethyl acetate solution was added to ethyl acetate (4 ml) solution of 83 mg of the above compound, and stirred for 3 hours. The precipitated solid was taken out through filtration, and washed with ethyl acetate to obtain 51 mg of 2-(2-amino-6-piperidin-3-ylpyrimidin-4-yl)-3-ethylphenol dihydrochloride as a pale yellow powder.

Example 74:

(1) A mixture solution of 200 mg of tert-butyl 3-[2-amino-6-(2,6-dihydroxyphenyl)pyrimidin-4-yl]piperidine-1-carboxylate, 254 mg of cesium carbonate and 4 ml of DMF was stirred at room temperature for 15 minutes, and then 112

mg of ethyl 2-bromo-2-methylpropanoate was added thereto and stirred at 60˚C for 18 hours. Next, 254 mg of cesium carbonate and 112 mg of ethyl 2-bromo-2-methylpropanoate were further added thereto, and stirred at 60˚C for 14 hours. 20 ml of water was added to the reaction mixture, extracted twice with 30 ml of ethyl acetate, and the extract was washed twice with 30 ml of water. The resulting organic layer was dried with anhydrous magnesium sulfate, and the solvent was evaporated to obtain 106 mg of crude tert-butyl 3-{2-amino-6-[2-(2-ethoxy-1,1-dimethyl-2-oxoethoxy)-6-hydroxyphenyl]pyrimidin-4-yl}piperidine-1-carboxylate as a yellow oil.
(2) Next, a mixture of 155 mg of the compound, 39 mg of lithium hydroxide monohydrate and 6 ml of ethanol/THF/water mixture (4/1/1) was stirred at room temperature for 4 hours. Next, 50 mg of lithium hydroxide monohydrate was added thereto, and stirred at room temperature for 17 hours. 5 ml of water was added to the reaction mixture, and then the mixture was neutralized with aqueous 1 M hydrogen chloride solution, and the resulting product was extracted with 30 ml of ethyl acetate and 40 ml of chloroform in that order. The extracts were combined, then dried with anhydrous magnesium sulfate, and the solvent was evaporated. The resulting residue was purified through silica gel column chromatography (chloroform) to obtain 106 mg of 2-(2-{2-amino-6-[1-(tert-butoxycarbonyl)piperidin-3-yl]pyrimidin-4-yl}-3-hydroxyphenoxy)-2-methylpropanoic acid as a yellow powder.
(3) Next, 1 ml of 4 M hydrogen chloride-dioxane solution was added to dioxane (8 ml) solution of 100 mg of the above compound, and stirred at room temperature for 8 hours. Next, a few drops of water were added to the reaction mixture, and stirred at room temperature for 15 hours. The reaction mixture was concentrated, and the resulting solid was washed with ethyl acetate to obtain 86 mg of 2-[2-(2-amino-6-piperidin-3-ylpyrimidin-4-yl)-3-hydroxyphenoxy]-2-methylpropanoic acid dihydrochloride as a yellow powder.

Example 75:

(1) 104 mg of allyl bromide was added to a mixture of 300 mg of tert-butyl 3-[2-amino-6-(2,6-dihydroxyphenyl) pyrimidin-4-yl]piperidine-1-carboxylate, 138 mg of potassium carbonate and 5 ml of DMF, and stirred at 100˚C for 5 hours. The reaction mixture was concentrated under reduced pressure, 20 ml of chloroform was added thereto, and washed with 20 ml of aqueous saturated ammonium chloride solution. The resulting organic layer was dried with anhydrous magnesium sulfate, and the solvent was evaporated. The resulting residue was purified through silica gel column chromatography (chloroform/methanol solution = 20/1) to obtain 347 mg of tert-butyl 3-[2-amino-6-(2-allyloxy-6-hydroxyphenyl)pyrimidin-4-yl]piperidine-1-carboxylate as a yellow powder.
(2) Next, 320 mg of the compound was heated at 200˚C for 30 minutes. The resulting solid was washed with acetonitrile to obtain 111 mg of tert-butyl 3-[6-(3-allyl-2,6-dihydroxyphenyl)-2-aminopyrimidin-4-yl]piperidine-1-carboxylate as a yellow powder.
(3) Next, 0.5 ml of 4 M hydrogen chloride-dioxane solution was added to dioxane (4 ml) solution of 200 mg of the above compound, and stirred for 1 hour. The reaction mixture was concentrated, and the resulting solid was washed with ethyl acetate-ethanol to obtain 89 mg of 4-allyl-2-(2-amino-6-piperidin-3-ylpyrimidin-4-yl)benzene-1,3-diol dihydrochloride as a yellow powder.

Example 76:

(1) 100 mg of 10 % Pd/C was added to a solution of 200 mg of tert-butyl 3-[6-(3-allyl-2,6-dihydroxyphenyl)-2-aminopyrimidin-4-yl]piperidine-1-carboxylate in a mixture of 5 ml of ethyl acetate and 5 ml of methanol, and then stirred in a hydrogen atmosphere for 3 hours. The reaction mixture was filtered through Celite, and the organic solvent was evaporated under reduced pressure to obtain 220 mg of tert-butyl 3-[2-amino-6-(2,6-dihydro-3-propylphenyl)-pyrimidin-4-yl]piperidine-1-carboxylate as a yellow powder.
(2) Next, 0.5 ml of 4 M hydrogen chloride-dioxane solution was added to dioxane (4 ml) solution of 210 mg of the above compound, and stirred for 1 hour. The reaction mixture was concentrated, and the resulting solid was washed with acetonitrile to obtain 52 mg of 2-(2-amino-6-piperidin-3-ylpyrimidin-4-yl)-4-propylbenzene-1,3-diol dihydrochloride as a yellow powder.

Example 77:

(1) 16 mg of platinum oxide was added to a solution of 160 mg of 2-[2-amino-6-(6-methylpyridin-3-yl)pyrimidin-4-yl]phenol in 3 ml of acetic acid, and stirred overnight in a hydrogen atmosphere at room temperature. The reaction mixture was filtered through Celite, and concentrated under reduced pressure. 15 ml of water was added to the resulting residue, and this was controlled to have a pH of 8 with aqueous 1 N sodium hydroxide solution added thereto, and then extracted with 30 ml of chloroform. The resulting organic layer was dried with anhydrous sodium sulfate, and the solvent was evaporated, and the resulting residue was purified through silica gel column chromatography (chloroform/aqueous 10 % ammonia-methanol solution = 94/6 to 90/10) to obtain 44 mg of 2-[2-amino-6-(6-methylpiperidin-3-yl)pyrimidin-4-yl]phenol as a colorless powder.

(2) Next, 18 mg of fumaric acid was added to methanol (3 ml) solution of 44 mg of the above compound, and stirred at room temperature for 1 hour, and then the reaction mixture was concentrated. The resulting solid was recrystallized from acetonitrile-methanol to obtain 37 mg of 2-[2-amino-6-(6-methylpiperidin-3-yl)pyrimidin-4-yl]phenol hemifumarate as a pale yellow powder.

Example 78:

(1) Under ice-cooling, 19.78 g of sodium hydride was added to THF (500 ml) solution of 47.25 g of guanidine hydrochloride, and then stirred at room temperature for 2 hours. The reaction mixture was concentrated, 800 ml of ethanol and 61.90 g of tert-butyl 3-[3-(5-ethyl-2-hydroxyphenyl)-3-oxopropanoyl]piperidine-1-carboxylate were added thereto, and stirred at 70˚C for 15 hours. The reaction mixture was concentrated, 400 ml of water was added to the resulting residue, then neutralized with aqueous 1 M hydrochloric acid solution, and extracted twice with 800 ml of chloroform. The resulting organic layer was dried with anhydrous sodium sulfate, and the solvent was evaporated. The resulting residue was recrystallized from hexane-ethyl acetate and from ethyl acetate-ethanol to obtain 30.18 g of tert-butyl 3-[2-amino-6-(5-ethyl-2-hydroxyphenyl)pyrimidin-4-yl]piperidine-1-carboxylate as a yellow powder.

(2) Next, 800 ml of 2 M hydrogen chloride-dioxane solution was added to 40.88 g of the above compound, and stirred at room temperature for 15 hours. The reaction mixture was concentrated, and the resulting solid was washed with acetonitrile-methanol to obtain 25.40 g of 2-(2-amino-6-piperidin-3-ylpyrimidin-4-yl)-4-ethylphenol dihydrochloride as a yellow powder.

(3) Next, 1.64 g of the above compound was converted into its free base by adding thereto aqueous 1 M sodium hydroxide solution, and then extracted with chloroform. The resulting organic layer was dried with anhydrous magnesium sulfate, and the solvent was evaporated. The resulting residue was dissolved in 15 ml of THF, then 1.33 g of N-tert-butoxycarbonyl-D-phenylalanine, 676 mg of HOBt and 959 mg of WSC·HCl were added thereto, and stirred at room temperature for 18 hours. Water was added to the reaction mixture, then extracted with chloroform, and the resulting organic layer was washed with aqueous saturated sodium bicarbonate solution, aqueous 1 M hydrochloric acid solution and further with brine. The resulting organic layer was dried with anhydrous magnesium sulfate, and the solvent was evaporated to obtain 2.86 g of tert-butyl ((1R)-2-{3-[2-amino-6-(5-ethyl-2-hydroxyphenyl)pyrimidin-4-yl]piperidin-1-yl}-1-benzyl-2-oxoethyl)carbamate as a yellow oil.

(4) Next, 2.85 g of the above compound was dissolved in 20 ml of ethyl acetate, 20 ml of 4 M hydrogen chloride-ethyl acetate solution was added thereto, and stirred at room temperature for 12 hours. The precipitated solid was taken out through filtration, and washed with ethyl acetate and diethyl ether in that order to obtain 2.38 g of 2-(2-amino-6-{1-[(2R)-2-amino-3-phenylpropanoyl]piperidin-3-yl}pyrimidin-4-yl)-4-ethylphenol hydrochloride as a yellow solid.

(5) Next, 2.38 g of the above compound was suspended in 30 ml of THF, and 1.39 ml of triethylamine was added thereto and stirred at room temperature for 1 hour. 0.61 ml of phenyl isothiocyanate was added to the reaction mixture, and stirred at room temperature for 12 hours. The solvent was evaporated from the reaction mixture to obtain N-((1R)-2-{3-[2-amino-6-(5-ethyl-2-hydroxyphenyl)pyrimidin-4-yl]piperidin-1-yl}-1-benzyl-2-oxoethyl)-N'-phenylthiourea as a diastereomer mixture thereof. The mixture was separated and purified through silica gel column chromatography (silica gel 60 N [Kanto Chemical Catalog No. 37561-84, spherical, neutral]; chloroform/ethyl acetate = 3/1) to separately obtain 1.07 g of a less-polar (first eluted) compound as a yellow oil, and 1.17 g of a more-polar (later eluted) compound as a yellow oil.

(6) 1.16 g of the more-polar compound obtained in (5) was dissolved in 10 ml of THF, and 5 ml of TFA was added thereto and stirred at room temperature for 8 hours. The solvent was evaporated under reduced pressure, and the residue was neutralized with aqueous 1 M sodium hydroxide solution, and then extracted with chloroform. The extract was washed with brine, then dried with anhydrous magnesium sulfate, and the solvent was evaporated under reduced pressure. The resulting residue was dissolved in methanol, then 1 ml of 4 M hydrogen chloride-ethyl acetate solution was added thereto, and the solvent was evaporated. The resulting residue was recrystallized from methanol to obtain 172 mg of (-)-2-(2-amino-6-piperidin-3-ylpyrimidin-4-yl)-4-ethylphenol dihydrochloride as a yellow solid.

Example 79:

The less-polar compound obtained in Example 78(5) was processed in the same manner as in Example 78(6) to obtain (+)-2-(2-amino-6-piperidin-3-ylpyrimidin-4-yl)-4-ethylphenol dihydrochloride.

Compounds of Examples 80 to 147 were produced in the same manner as in the above Examples. Their structural formulae and physical properties are shown in Tables 16 to 22 below.
Compounds in Table 23 below could be readily produced almost in the same manner as in the above-mentioned Examples or the above-mentioned production methods, or according to modifications derived from those methods by modifying them in some degree in a mode obvious to those skilled in the art.
In the Reference Examples and in the Tables mentioned below, the abbreviations used have the meanings mentioned below. In the Tables, the number before the substituent indicates the substituent position; and "-" in the column "$(R^1)_n$" means that n in the compound is 0, or that is, the compound does not have the substituent.

Ex: Example Number,
REx: Reference Example Number,
Cmp: Compound Number,
Str: Structural Formula,
Syn: Production Method (the number indicates the Example Number that was referred to for producing the compound),
Dat: Physicochemical Data (F1: FAB-MS(M+H)$^+$, F2: FAB-MS(M-H)$^-$, E1: ESI-MS(M+H)$^+$, E2: ESI-MS(M-H)$^-$, E3: ESI-MS(M+Na)$^+$, A1: APCI-MS(M+H)$^+$, A2:
APCI-MS(M-H)$^-$, NMR1: $\delta$ (ppm) of characteristic peaks in $^1$H-NMR in $CDCl_3$, NMR2: $\delta$ (ppm) of characteristic peaks in $^1$H-NMR in DMSO-$d_6$),
Sal: (HCl: hydrochloride, 2HCl: dihydrochloride, Fum: fumarate, 0.5Fum: 1/2-fumarate, blank or hyphen: salt free),
Me: methyl,
Et: ethyl,
iPr: isopropyl,
nPr: n-propyl,
tBu: tert-butyl,
iBu: isobutyl,
cHex: cyclohexyl,
Ph: phenyl,
Bn: benzyl,
Boc: tert-butoxycarbonyl,
Ac: acetyl.

[Table 1]

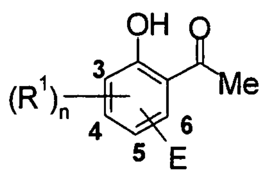

| Rex | $(R^1)_n$ | E | Dat | Rex | $(R^1)_n$ | E | Dat |
|---|---|---|---|---|---|---|---|
| 7 | - | 6- 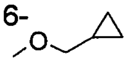 | F2:205 | 10 | 3-Et | 6- 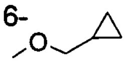 | A2:221 |
| 8 | - | 4- 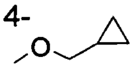 | F2:205 | 11 | 5-Et | 4- 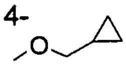 | E2:221 |
| 9 | - | 5- 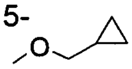 | F2:205 | 12 | 3-Me | 4- 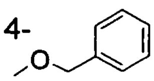 | F1:257 |

[Table 2]

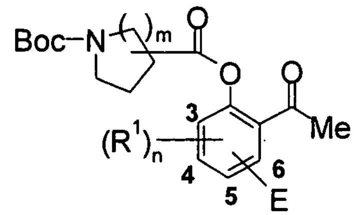

| Rex | $(R^1)_n$ | E | 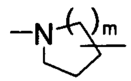 | Dat | Rex | $(R^1)_n$ | E | 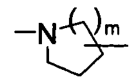 | Dat |
|---|---|---|---|---|---|---|---|---|---|
| 13 | 5-F | H | 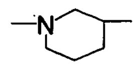 | F1:366 | 23 | 5-tBu | H | 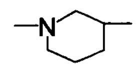 | F1:404 |
| 14 | 4-F | H | 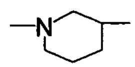 | F1:366 | 24 | 3-Me, 5-Me | H | 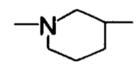 | F1:376 |
| 15 | 5-Me | H | 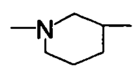 | F1:362 | 25 | 5-Et | H | 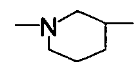 | F1:376 |
| 16 | - | H | 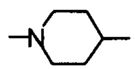 | F1:348 | 26 | 5-iPr | H | 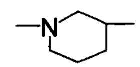 | E1:390 |
| 17 | 5-Me | H | 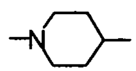 | F1:362 | 27 | - | 5-cHex | 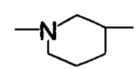 | E1:430 |
| 18 | 5-Me | H | 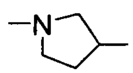 | F1:348 | 28 | 4-Me | 6- 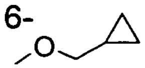 | 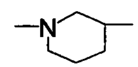 | F1:432 |
| 19 | 5-Me | H | 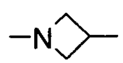 | F1:334 | 29 | - | 4- 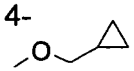 | 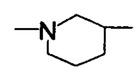 | F1:418 |
| 20 | 5-Me | H | 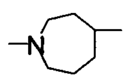 | E3:398 | 30 | 3-Me | 4- 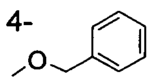 | 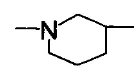 | F1:468 |

(continued)

| Rex | $(R^1)_n$ | E | 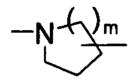 | Dat | Rex | $(R^1)_n$ | E | 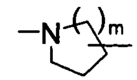 | Dat |
|---|---|---|---|---|---|---|---|---|---|
| 21 | - | 6- 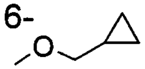 | 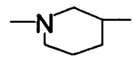 | F1:418 | 31 | - | 5- 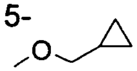 | 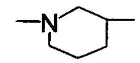 | F1:418 |
| 22 | - | 6- 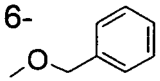 | 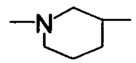 | F1:454 | | | | | |

[Table 3]

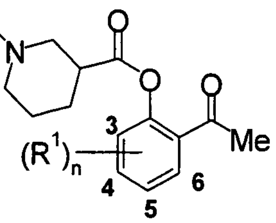

| Rex | $(R^1)_n$ | Dat |
|---|---|---|
| 32 | 4-Me, 5-Me | NMR1:3.19 (1H, m), 6.84 (1H, s), 7.57 (1H, s) |
| 33 | 5-Cl | NMR1:3.90 (1H, m), 7.05 (1H, d, J=8.8), 7.74 (1H, d, J=2.4) |
| 34 | 5-MeO | NMR1:3.18 (1H, m), 6.99 (1H, d, J=8.8), 7.28 (1H, d, J=2.8) |
| 35 | 4-MeO | NMR1:3.21 (1H, m), 6.58 (1H, d, J=2.4), 7.82 (1H, d, J=8.8) |
| 36 | 3-Cl, 5-Cl | NMR1:3.12 (1H, m), 7.60 (1H, d, J=2.4), 7.65 (1H, d, J=2.4) |
| 37 | 5-Br | NMR1:2.93 (1H, m), 6.99 (1H, d, J=8.4), 7.89 (1H, d, J=2.4) |
| 38 | 5-Cl, 4-Me | NMR1:2.94 (1H, m), 6.97 (1H, s), 7.78 (1H, s) |
| 39 | 4-Me | NMR1:2.92 (1H, m), 6.89 (1H, s), 7.72 (1H, d, J=8.0) |
| 40 | 3-Br, 5-Br | NMR1:3.11 (1H, m), 7.82 (1H, d, J=2.4), 7.90 (1H, d, J=2.0) |
| 41 | 3-Br, 5-Cl | NMR1:3.11 (1H, m), 7.69 (1H, d, J=2.4), 7.76 (1H, d, J=2.4) |
| 42 | 5-MeS | NMR1: 7.02 (1H, d, J=8.8), 7.39 (1H, dd, J=8.8, J=2.0), 7.64 (1H, d, J=2.0) |

[Table 4]

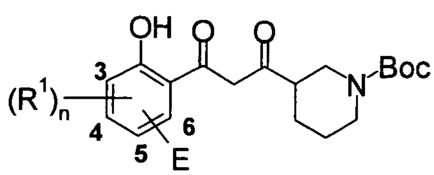

| Rex | $(R^1)_n$ | E | Dat | Rex | $(R^1)_n$ | E | Dat | Rex | $(R^1)_n$ | E | Dat |
|---|---|---|---|---|---|---|---|---|---|---|---|
| 43 | 5-F | H | F1:366 | 52 | 5-Cl, 4-Me | H | E2:394 | 61 | - | 6- 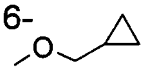 | F2:416 |
| 44 | 4-F | H | F1:366 | 53 | 4-Me | H | E2:360 | | | | |
| 45 | 5-Me | H | F1:362 | 54 | 3-Br, 5-Br | H | E2:504 | 62 | - | 6- 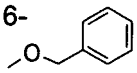 | E2:452 |
| 46 | 4-Me, 5-Me | H | E2:374 | 55 | 3-Br, 5-Cl | H | A2:460 | | | | |
| 47 | 5-Cl | H | E2:380 | 56 | 5-tBu | H | E2:402 | 63 | - | 5-cHex | F1:430 |
| 48 | 5-MeO | H | E2:376 | 57 | 3-Me, 5-Me | H | F1:376 | 64 | 4-Me | 6- 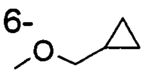 | F1:432 |
| 49 | 4-MeO | H | E2:376 | 58 | 5-Et | H | F1:376 | | | | |

(continued)

| Rex | $(R^1)_n$ | E | Dat | Rex | $(R^1)_n$ | E | Dat | Rex | $(R^1)_n$ | E | Dat |
|---|---|---|---|---|---|---|---|---|---|---|---|
| 50 | 3-Cl, 5-Cl | H | E2:414 | 59 | 5-iPr | H | F1:390 | 65 | - | 5- 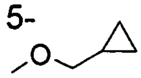 | F1:418 |
| 51 | 5-Br | H | E2:426 | 60 | 5-MeS | H | E1:393 | | | | |

[Table 5]

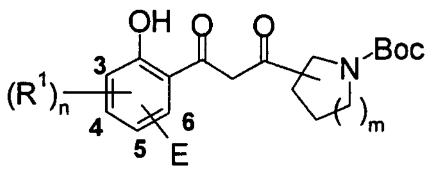

| Rex | $(R^1)_n$ | E | 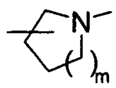 | Dat | Rex | $(R^1)_n$ | E | | Dat |
|---|---|---|---|---|---|---|---|---|---|
| 66 | - | H | 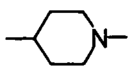 | F1:348 | 71 | - | 4- 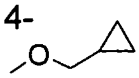 | 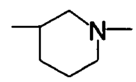 | NMR1: 6.39 (1H, d, J=2.4), 6.45 (1H, dd, J=8.8, J=2.4), 7.64 (1H, d, J=8.8) |
| 67 | 5-Me | H | 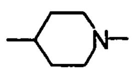 | F1:362 | | | | | |
| 68 | 5-Me | H | 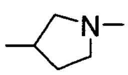 | F2:346 | 72 | 3-Me | 4- 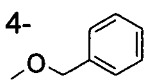 | 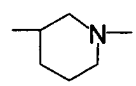 | NMR1: 5.13 (2H, s), 6.82 (1H, d, J=8.8), 7.69 (1H, d, J=8.8) |
| 69 | 5-Me | H | 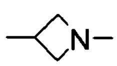 | F1:334 | | | | | |
| 70 | 5-Me | H | 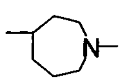 | F1:376 | | | | | |

[Table 6]

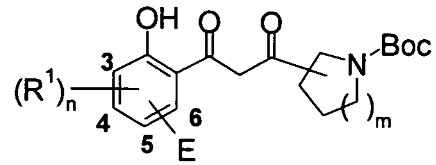

| Rex | $(R^1)_n$ | E | 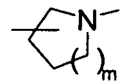 | Dat | Rex | $(R^1)_n$ | E | 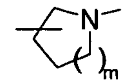 | Dat |
|---|---|---|---|---|---|---|---|---|---|
| 73 | 5-nPr | H | 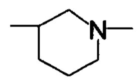 | F1:348 | 75 | 5-Et | 4- 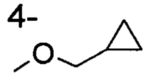 | 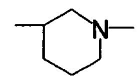 | F2:444 |
| 74 | 3-Et | 6- 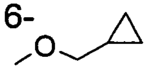 | 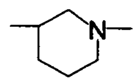 | F2:444 | | | | | |

[Table 7]

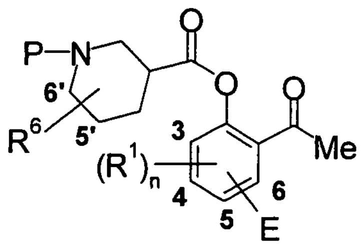

| Rex | P | $R^6$ | $(R^1)_n$ | E | Dat | Rex | P | $R^6$ | $(R^1)_n$ | E | Dat |
|---|---|---|---|---|---|---|---|---|---|---|---|
| 78 | Boc | 6'-$CH_2CH_2Ph$ | 5-Me | H | E3:488 | 81 | Boc | 6'-Me | - | 6-OBn | F1:468 |
| 79 | Boc | 5'-Me | | H | F1:362 | 82 | Bn | H | 5-Me | H | F1:352 |
| 80 | Boc | H | 4-Br | H | F1:426 | 83 | Boc | 5'-Me | 5-Me | H | F1:376 |

[Table 8]

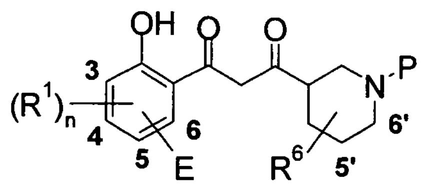

| Rex | P | $R^6$ | $(R^1)_n$ | E | Dat | Rex | P | $R^6$ | $(R^1)_n$ | E | Dat |
|---|---|---|---|---|---|---|---|---|---|---|---|
| 84 | Boc | 6'-$CH_2CH_2Ph$ | 5-Me | H | E2:464 | 87 | Boc | 6'-Me | - | 6-OBn | F1:468 |
| 85 | Boc | 5'-Me | - | H | F1:362 | 88 | Bn | H | 5-Me | H | F1:352 |
| 86 | Boc | H | 4-Br | H | F1:426 | 89 | Boc | 5'-Me | 5-Me | H | F1:376 |

[Table 9]

| Rex | Str | Dat | Rex | Str | Dat |
|---|---|---|---|---|---|
| 90 | 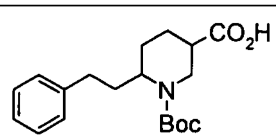 | E2:332 | 93 | 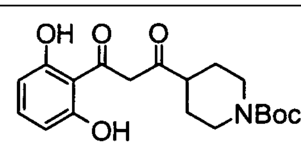 | F1:364 |
| 91 | 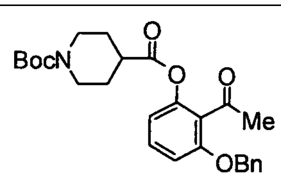 | F1:454 | 94 | 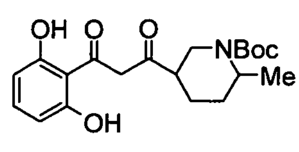 | F1:378 |
| 92 | 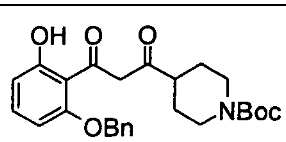 | F1:454 | | | |

[Table 10]

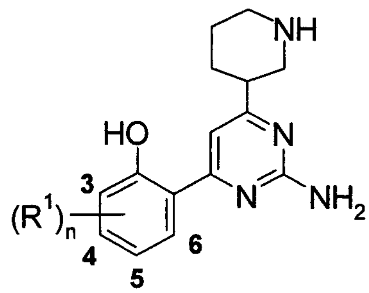

| Ex | Syn | $(R^1)_n$ | Sal | Dat |
|---|---|---|---|---|
| 1 | 1 | H | 2HCl | F1:271, NMR2:2.80-2.93 (1H, m), 6.90-7.01(2H, m), 8.01 (1H, dd, J=8.3, 1.5Hz) |
| 12 | 1 | 5-F | 2HCl | F1:289, NMR2:2.79-2.93 (1H, m), 7.43(1H, s), 7.91 (1H, dd, J=10.3, 2.9Hz) |
| 13 | 1 | 4-F | 2HCl | F1:289, NMR2:2.80-2.92 (1H, m), 7.34(1H, s), 8.11 (1H, dd, J=9.7,6.9Hz) |
| 14 | 1 | 5-Cl | 2HCl | F1:305, NMR2:2.80-2.84 (1H, m), 7.00 (1H, d, J=8.8Hz), 7.44 (1H, s) |
| 15 | 1 | 3-Cl, 5-Cl | HCl | F1:339, NMR2:3.38-3.43 (1H, m), 7.47 (1H, s), 8.18 (1H, d, J=2.9Hz) |
| 16 | 1 | 3-Br, 5-Cl | HCl | F1:385, NMR2:2.85-2.89 (1H, m), 7.46 (1H, s), 7.80 (1H, d, J=2.9Hz) |
| 17 | 1 | 5-Br | 2HCl | F1:351, NMR2:2.84-2.88 (1H, m), 6.98 (1H, d, J=8.7Hz), 7.47 (1H, s) |
| 18 | 1 | 3-Br, 5-Br | HCl | F1:429, NMR2:2.85-2.89 (1H, m), 7.46 (1H, s), 7.90 (1H, d, J=2.2Hz) |
| 19 | 1 | 5-Cl-4-Me | 2HCl | F1:319, NMR2:2.80-2.84 (1H, m), 7.01 (1H, s), 7.46 (1H,s) |
| 20 | 1 | 3-Me-4-OH | 2HCl | E1:301, NMR2:6.51 (1H, d, J=8.7Hz), 7.20 (1H, s), 7.76 (1H, d, J=8.7Hz) |
| 21 | 1 | 4-Me | 2HCl | F1:285, NMR2:2.85-2.92 (1H, m), 7.32 (1H, s), 7.90 (1H, d, J=8.1Hz) |
| 22 | 1 | 5-Me | 2HCl | F1:285, NMR2:2.29(3H, s), 2.80-2.88 (1H, m), 7.41 (1H, s) |
| 23 | 1 | 5-Et | 2HCl | E1:299, NMR2:1.20 (3H, t, J=7.6), 6.92 (1H, d, J=8.3Hz), 7.83 (1H, d, J=2.0Hz) |
| 24 | 1 | 4-Me, 5-Me | 2HCl | F1:299, NMR2:2.84-2.88 (1H, m), 7.34 (1H, s), 7.79 (1H, s) |
| 25 | 1 | 3-Me, 5-Me | 2HCl | E1:299, NMR2:2.17 (3H, s), 2.26 (3H, s), 7.70 (1H, s) |

[Table 11]

| | | | | |
|---|---|---|---|---|
| 26 | 1 | 5-MeO | 2HCl | F1:301, NMR2:2.80-2.95 (1H, m), 3.79 (3H, s), 6.93 (1H, d, J=8.8Hz) |
| 27 | 1 | 4-MeO | 2HCl | F1:301, NMR2:2.80-2.90 (1H, m), 3.81 (3H, s), 7.33 (1H, s) |
| 28 | 1 | 5-MeS | 2HCl | F1:317, NMR2:7.40 (1H, dd, J=8.6, 2.5Hz), 7.44 (1H, s), 7.93 (1H, d, J=2.5Hz) |
| 29 | 1 | 5-tBu | 2HCl | F1:327, NMR2:2.81-2.95 (1H, m), 6.96 (1H, d, J=8.6Hz), 7.46 (1H, dd, J=2.5, 8.6Hz) |

(continued)

| | | | | |
|---|---|---|---|---|
| 30 | 1 | 5-iPr | 2HCl | F1:313, NMR2:7.30 (1H, dd, J=8.8, 2.0Hz), 7.39 (1H, s), 7.81 (1H, d, J=2.0Hz) |
| 31 | 1 | 5-nPr | 2HCl | F1:313, NMR2:6.88 (1H, d, J=8.3Hz), 7.23 (1H, dd, J=8.3, 1.9Hz), 7.37 (1H, s) |

[Table 12]

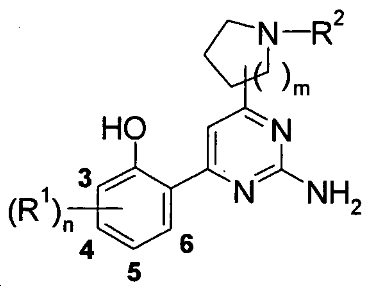

| Ex | Syn | $(R^1)_n$ | 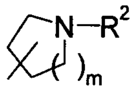 | Sal | Dat |
|---|---|---|---|---|---|
| 2 | 2 | - | 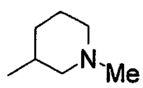 | | F1:285, NMR2:2.22 (1H, s), 7.19(1H, s), 13.95 (1H,s) |
| 32 | 1 | - | 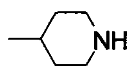 | 2HC1 | F1:271, NMR2:2.93-3.08 (3H,m), 7.05 (1H, dd, J= 8.3, 1.0Hz), 7.35(1H, s) |
| 33 | 1 | 5-Me | 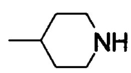 | 2HCl | F1:285, NMR2:2.92-3.06 (3H, m), 6.87 (1H, d, J=8.3Hz), 7.32(1H, s) |
| 34 | 1 | 5-Me | 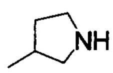 | 2HCl | E1:271, NMR2:2.06-2.15 (1H, m), 6.87 (1H, d, J=8.0Hz), 7.22 (1H, dd, J=8.4, 1.6Hz) |
| 35 | 1 | 5-Me | 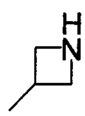 | 2HC1 | F1:257, NMR2:4.08-4.28 (5H, m), 6.85 (1H, d, J=8.0Hz), 7.21 (1H, dd, J=8.4, 1.6Hz) |
| 36 | 1 | 5-Me | 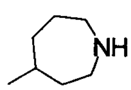 | 2HCl | F1:299, NMR2:1.82-1.90 (2H, m), 6.86 (1H, d, J=8.4Hz), 7.22 (1H, d, J=8.4Hz) |

[Table 13]

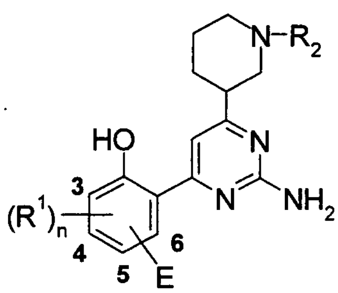

(continued)

| Ex | Syn | $(R^1)_n$ | E | $R^2$ | Sal | Dat |
|---|---|---|---|---|---|---|
| 3 | 3 | - | 6-Bn-O- | $-CO_2$-tBu | - | E1:477, NMR2:3.88-3.95 (1H, m), 5.13 (2H, s), 6.53 (1H, d, J=8.4Hz) |
| 4 | 4 | 6-OH | H | $-CO_2$-tBu | - | E1:387, NMR2:3.86-3.93 (1H, m), 6.47 (2H, d, J=8.4Hz), 7.16 (1H, t, J=8.4Hz) |
| 5 | 5 | 6-EtO | H | H | 2HCl | F1:315, NMR2:6.60 (1H, d, J=8.3Hz), 6.64 (1H, d, J=8.3Hz), 7.29 (1H, s) |
| 37 | 1 | - | 6- 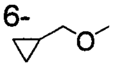 | H | 2HCl | F1:341, NMR2:2.81-2.93 (1H, m), 6.57 (1H, d, J=8.3Hz), 7.30 (1H, t, J=8.3Hz) |
| 38 | 1 | - | 5- 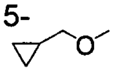 | H | 2HCl | F1:341, NMR2:6.91 (1H, d, J=9.3Hz), 7.43 (1H, s), 7.49 (1H, d, J=3.5Hz) |
| 39 | 1 | - | 4- 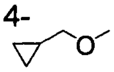 | H | 2HCl | F1:341, NMR2:6.49 (1H, d, J=2.5Hz), 7.33 (1H, s), 7.99 (1H, d, J=9.3Hz) |
| 40 | 1 | 4-Me | 6- 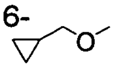 | H | 2HCl | F1:355, NMR2:6.41 (1H, s), 6.45 (1H, s), 7.41 (1H, s) |
| 41 | 1 | 3-Et | 6- 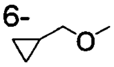 | H | 2HCl | F1:369, NMR2:6.48 (1H, d, J=8.3Hz), 7.17 (1H, d, J=8.3Hz), 7.63 (1H, s) |
| 42 | 1 | 5-Et | 4- 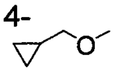 | H | 2HCl | F1: 369, NMR2:6.46 (1H, s), 7.28 (1H, s), 7.78 (1H, s) |
| 43 | 1 | - | 5-cHex | H | 2HCl | F1:353, NMR2:6.93 (1H, d, J=8.3Hz), 7.44 (1H, s), 7.80 (1H, d, J=1.9Hz) |
| 44 | 5 | 6-MeO | H | H | 2HCl | F1:301, NMR2:3.80 (3H, s), 6.67 (1H, d, J=8.3Hz), 7.21 (1H, s) |
| 45 | 5 | - | 6 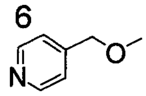 | H | 3HCl | E1:378, NMR2:7.28 (1H, s), 8.04 (2H, d, J=6.3Hz), 8.99 (2H, d, J=6.3Hz) |
| 46 | 5 | - | 6- 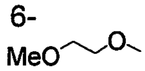 | H | 2HCl | F1:345, NMR2:3.34 (3H, s), 7.29 (1H, t, J=8.3Hz), 7.45 (1H, s) |
| 47 | 5 | - | 6-Bn-O- | H | 2HCl | F1:377, NMR2:5.13 (2H, s), 6.68 (1H, d, J=8.3Hz), 6.75 (1H, d, J=8.8Hz) |
| 48 | 5 | - | 6- 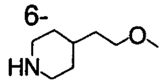 | H | 3HCl | E1:398, NMR2:6.66 (1H, d, J=8.3Hz), 7.25 (1H, s), 7.31 (1H, t, J=8.3Hz) |

[Table 14]

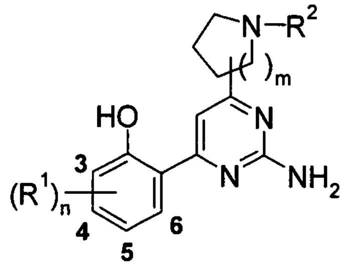

| Ex | Syn | $(R^1)_n$ | 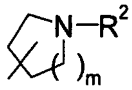 | Sal | Dat |
|---|---|---|---|---|---|
| 6 | 6 | 5-Me | 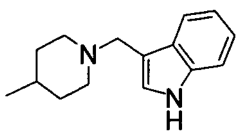 | 2HCl | E1:414, NMR2:2.26 (3H, s), 3.47-3.58(2H, m), 7.06-7.28 (4H, m) |
| 7 | 7 | 5-Me | 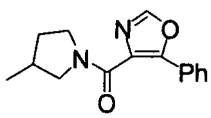 | HCl | F1:442, NMR2:2.14-2.33 (5H, m), 6.81-6.85 (1H, m), 7.42-7.51 (3H, m) |
| 8 | 8 | 5-Et | 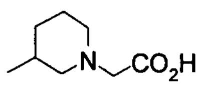 | 2HCl | F1:357, NMR2:1.20 (3H, t, J=7.6Hz), 4.19 (2H, s), 7.76-7.82 (1H, m) |
| 9 | 9 | 5-Me | 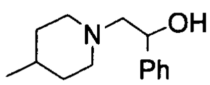 | 2HCl | E1:405, NMR2:2.29 (3H, s), 3.74-3.86(2H, m), 6.88-6.94 (1H, m) |
| 10 | 10 | 6-OH | 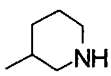 | 2HCl | F1:287, NMR2:2.83-2.93 (1H, m), 6.52 (2H, d, J=8.3Hz), 7.16 (1H, t, J=8.3Hz) |
| 11 | 11 | 5-Me | 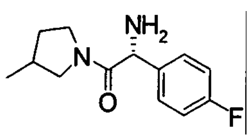 | 2HCl | F1:422, NMR2:2.01-2.33 (5H, m), 5.35-5.39 (1H, m), 6.89 (1H, dd, J=8.3, 2.0Hz) |
| 49 | 1 | 5-Me | 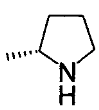 | 2HCl | E1:271, NMR2:3.30-3.39 (2H, m), 6.84 (1H, d, J=8.3Hz), 7.21 (1H, dd, J=8.3, 2.0Hz) |
| 50 | 1 | 5-Me | 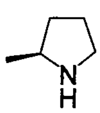 | 2HCl | F1:271, NMR2:2.00-2.05 (3H, m), 6.83 (1H, d, J=8.4Hz), 7.20 (1H, dd, J=8.3, 2.0Hz) |
| 51 | 2 | - | 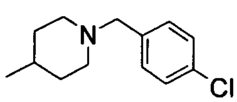 | 2HCl | F1:395, NMR2:2.09-2.23 (4H, m), 7.74-7.80(2H, m), 7.93-8.08(1H,m) |
| 52 | 2 | - | 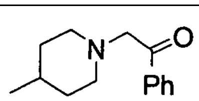 | 2HCl | F1:389, NMR2:2.98-3.10 (1H, m), 6.94-7.00(1H, m), 7.98-8.04 (2H, m) |

(continued)

| Ex | Syn | $(R^1)_n$ | 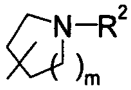 | Sal | Dat |
|---|---|---|---|---|---|
| 53 | 2 | 5-Me | 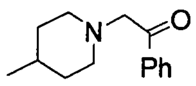 | 2HCl | E1:403, NMR2:2.29 (3H, s), 3.64-3.76(2H, m), 7.34 (1H, s) |
| 54 | 2 | 5-Et | 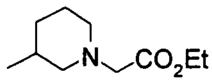 | | F1:385, NMR2:1.19 (6H, t, J=7.4Hz), 4.09 (2H, q, J=7.2Hz), 7.31 (1H, s) |

[Table 15]

| | | | | | |
|---|---|---|---|---|---|
| 55 | 7 | 5-Me | 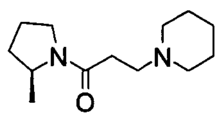 | 2HCl | E1:410, NMR2 : 1.28-1.42 (1H, m), 6.91 (1H, d, J=8.3Hz), 7.26 (1H, dd, J=8.3, 1.5Hz) |
| 56 | 7 | 5-Me | 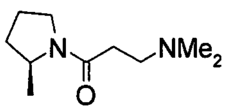 | 2HCl | E1:370, NMR2 : 1.85-2.06 (4H, m), 6.83-6.89 (1H, m), 7.18-7.25 (1H, m) |
| 57 | 7 | 5-Me | 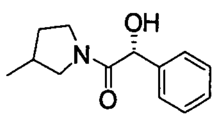 | HCl | F1:405, NMR2 : 2.06-2.27 (5H, m), 5.23-5.28 (1H, m), 6.85 (1H, d, J=8.4Hz) |
| 58 | 7 | 5-Me | 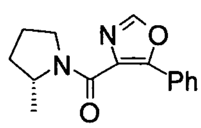 | HCl | F1:442, NMR2 : 1.92-2.02 (3H, m), 3.76-3.96 (2H, m), 7.79-7.81 (1H, m) |
| 59 | 7 | 5-Me | 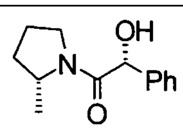 | HCl | F1:405, NMR2 : 1.77-1.91 (3H, m), 4.84 (1H, dd, J=8.3, 5.1Hz), 6.85 (1H, d, J=8.3Hz) |
| 60 | 7 | 5-Me | 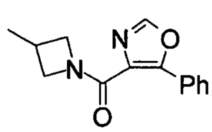 | HCl | F1:428, NMR2 : 4.67 (1H, dd, J=9.7, 6.3), 6.87 (1H, d, J=8.3Hz), 7.22 (1H, dd, J=8.3, 1.5Hz) |
| 61 | 7 | 5-Me | 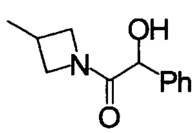 | HCl | F1: 391, NMR2 : 4.32-4.49 (3H, m), 5.10 (1H, d, J=9.2Hz), 6.84 (1H, d, J=8.4Hz) |
| 62 | 11 | 5-Me | 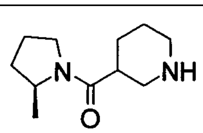 | 2HCl | E1:382, NMR2 : 2.84-3.14 (5H, m), 4.81-4.87 (1H, m), 6.82-6.92 (1H, m) |
| 63 | 11 | 5-Me | 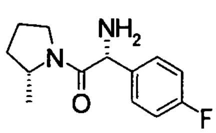 | 2HCl | F1: 422, NMR2 : 1.76-1.82 (1H, m), 3.87-3.93 (1H, m), 6.85 (1H, d, J=8.3Hz) |

(continued)

| | | | | | |
|---|---|---|---|---|---|
| 64 | 11 | 5-Me | 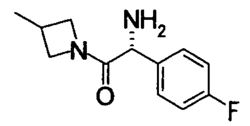 | 2HCl | F1:408, NMR2 : 4.53-4.57 (1H, m), 5.23-5.28 (1H,m), 6.82-6.86 (1H, m) |

[Table 16]

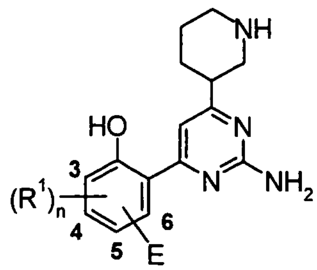

| Ex | Syn | $(R^1)_n$ | E | Sal | Dat |
|---|---|---|---|---|---|
| 65 | 65 | - | 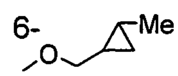 | 2HCl | F1:355, NMR2:0.29-0.37(1H, m), 1.03 (3H, d, J=5.9Hz), 7.38(1H, s) |
| 66 | 66 | - | 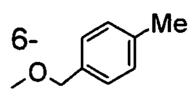 | Fum | F1:391, NMR2:2.33(3H, s), 6.52(1H, d, J=8.3Hz), 7.41(2H, d, J=7.8Hz) |
| 67 | 67 | 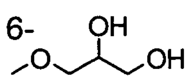 | H | Fum | E1:361, NMR2:1.62-1.89(3H, m), 3.45-3.57(2H, m), 6.48-6.56(4H, m) |
| 68 | 68 | 5-SOMe | H | 2HCl | E1:333, NMR2:7.13(1H, d, J=8.8Hz), 7.39(1H, s), 8.28(1H, d, J=2.0Hz) |
| 69 | 69 | 5-$SO_2$Me | H | 2HCl | F1:349, NMR2:7.14(1H, d, J=8.8Hz), 7.42(1H, s), 8.48(1H, d, J=2.4Hz) |
| 70 | 70 | 6-CH=$CH_2$ | H | 2HCl | E1:297, NMR2:5.24(1H, d, J=11.2Hz), 5.75(1H, d, J=16.8Hz), 7.30(1H, t, J=8.4Hz) |
| 71 | 71 | - | 6-NHBn | 3HCl | F1:376, NMR2:4.29(2H, s), 6.07(1H, d, J=8.8Hz), 7.02(1H, t, J=8.0Hz) |
| 72 | 72 | 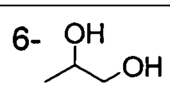 | H | 2HCl | F1:331, NMR2:4.41-4.45(1H, m), 7.04 (1H, d, J=8.0Hz), 7.30(1H, t, J=8.0Hz) |
| 73 | 73 | 6-Et | H | 2HCl | F1:299, NMR2:1.04(3H, t, J = 8.0Hz), 2.44(2H, q, J=7.6Hz), 7.25(1H, t, J=8.0Hz) |
| 74 | 74 | 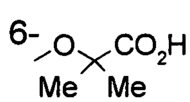 | H | 2HCl | F1:373, NMR2: 2.80-2.96 (1H, m), 3.06-3.30 (3H, m), 6.33 (1H, d, J=B. 0Hz) |
| 75 | 75 | 3-O-Allyl, 6-OH | H | 2HCl | F1:327, NMR2:5.88-6.00 (1H, m), 6.46 (1H, d, J=8.4Hz), 7.62 (1H, s) |
| 76 | 76 | 3-riPr, 6-OH | H | 2HCl | F1:329, NMR2:0.89 (3H, t, J=7.6Hz), 6.39(1H, d, J=8.0Hz), 7.61 (1H, s) |

(continued)

| Ex | Syn | $(R^1)_n$ | E | Sal | Dat |
|---|---|---|---|---|---|
| 80 | 1 | 4-Br | H | 2HCl | F1:349, NMR2:3.02-3.31 (3H, m), 3.36-3.45 (1H, m), 7.97 (1H, d, J=8.8Hz) |
| 81 | 5 | 6-iBuO | H | 2HCl | F1:343, NMR2: 0.92(6H, d, J=6.9Hz), 3.79(2H, dd, J=6.3, 1.2Hz), 7.24(1H, s) |
| 82 | 5 | - | 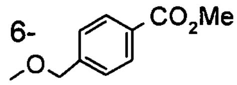 | 2HCl | F1:435, NMR2: 3.86(3H, s), 7.24(1H, s), 7.64(2H, d, J=7.8Hz) |

[Table 17]

| | | | | | |
|---|---|---|---|---|---|
| 83 | 5 | - | 6-O-$CH_2$-cHex | 2HCl | F1:383, NMR2:2.00-2.09 (1H, m), 2.81-2.97 (1H, m), 6.57-6.62 (2H, m) |
| 84 | 5 | 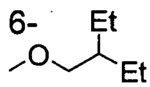 | H | 2HCl | F1:371, NMR2:1.55-1.71 (2H, m), 1.96-2.09 (1H, m), 6.56-6.67 (2H, m) |
| 85 | 5 | - | 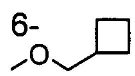 | 2HCl | F1:355, NMR2:2.80-2.94 (1H, m), 3.05-3.3.30 (3H, m), 6.58-6.68 (2H, m) |
| 86 | 5 | 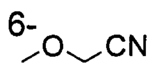 | H | 2HCl | E1:326, NMR2:5.23(2H, s), 6.63-6.67(2H, m), 7.01(1H, s) |
| 87 | 5 | 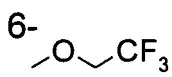 | H | 2HCl | F1:369, NMR2:4.83(2H, q, J=8.8Hz), 6.61-6.64(2H, m), 7.21(1H, s) |
| 88 | 5 | 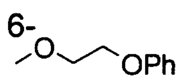 | H | 2HCl | F1:407, NMR2:4.32-4.40(4H, m), 6.66-6.70 (2H, m), 6.92-6.98(3H, m) |
| 89 | 5 | 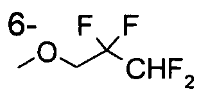 | H | 2HCl | F1:401, NMR2:4.05(2H, t, J=14Hz), 6.44-6.73(1H, m), 7.15(1H, m) |
| 90 | 5 | - | 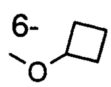 | 2HCl | F1:341, NMR2:4.69-4.77(1H, m), 6.42(1H, d, J=8.4Hz), 7.31(1H, m) |
| 91 | 5 | 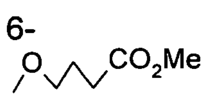 | H | 2HCl | F1:401, NMR2:1.98-2.10 (1H, m), 2.81-2.95 (1H, m), 7.24-7.33 (2H, m) |
| 92 | 65 | - | 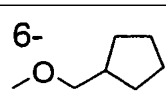 | 2HCl | F1:369, NMR2:3.58(2H, d, J=6.9Hz), 7.44 (1H, s), 7.21(1H, t, J=8.3Hz) |
| 93 | 65 | - | 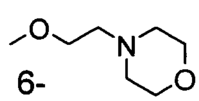 | 3HCl | E1:400, NMR2:4.48(2H, t, J=4.3Hz), 7.19 (1H, s), 7.33(1H, t, J=8.3Hz) |
| 94 | 65 | 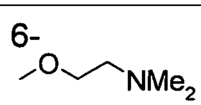 | H | 3HCl | E1:358, NMR2:2.78(6H, s), 7.26(1H, s), 7.35 (1H, t, J=8.3Hz) |

(continued)

| | | | | | |
|---|---|---|---|---|---|
| 95 | 65 | - | 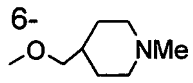 | 3HCl | F1:398, NMR2:3.86-3.96(2H, m), 6.56-6.65 (2H, m), 7.22-7.61(2H, m) |
| 96 | 65 | 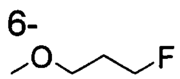 | H | 2HCl | F1:347, NMR2:2.80-2.94 (1H, m), 3.08-3.32 (3H, m), 7.31 (1H,t,J=8.3Hz) |
| 97 | 65 | - | 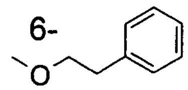 | 2HCl | F1:391, NMR2:4.25(2H, t, J=6.4Hz), 6.62-6.66(2H, m), 7.08(1H, s) |
| 98 | 65 | - | 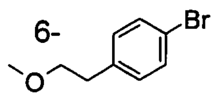 | 2HCl | F1:471, 469, NMR2:4.20-4.28(2H, m), 7.09 (1H, s), 7.43(2H, d, J=8.3Hz) |
| 99 | 65 | - | 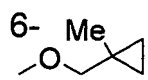 | 2HCl | F1:355, NMR2:0.38-0.44(2H, m), 1.15(3H, s), 6.52(1H, d, J=8.8Hz) |

[Table 18]

| | | | | | |
|---|---|---|---|---|---|
| 100 | 65 | 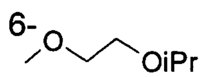 | H | 2HCl | F1:373, NMR2:1.05-1.09(6H, m), 4.09-4.15 (2H, m), 6.40 (1H, d, J=8.6Hz) |
| 101 | 65 | 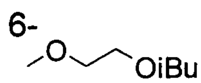 | H | 2HCl | F1:387, NMR2:0.79(6H, d, J=6.8Hz), 6.60-6.66(2H, m), 7.28-7.33(2H, m) |
| 102 | 65 | - | 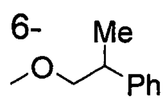 | 2HCl | F1:405, NMR2:7.00(3H, d, J=7.6Hz), 4.14(2H, d, J=4.8Hz), 6.60-6.65(2H, m) |
| 103 | 65 | - | 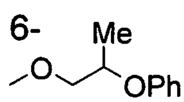 | 2HCl | F1:421, NMR2:1.29-1.34(3H, m), 6.60-6.66(2H, m), 6.90-6.95(3H, m) |
| 104 | 65 | - | 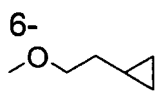 | 2HCl | F1:355, NMR2:0.06-0.11(2H, m), 0.38-0.45(2H, m), 7.26-7.33(2H, m) |
| 105 | 65 | 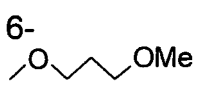 | H | 2HCl | F1:359, NMR2:3.22(3H, s), 7.27(1H, s), 7.30(1H, t, J=8.4Hz) |
| 106 | 65 | 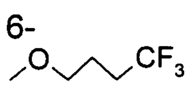 | H | 2HCl | F1:397, NMR2:4.08(2H, t, J=6.0Hz), 7.22 (1H, s), 7.32(1H, t, J=8.4Hz) |
| 107 | 65 | 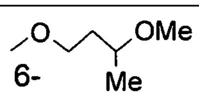 | H | 2HCl | E1:373, NMR2:1.08-1.09(3H, m), 4.02-4.10(2H, m), 6.61-6.67(2H, m) |
| 108 | 66 | - | 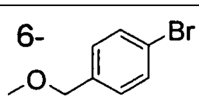 | Fum | F1:457, NMR2:5.12(2H, s), 7.50(2H, d, J=8.3Hz), 7.65(2H, d, J=8.3Hz) |
| 109 | 66 | - | 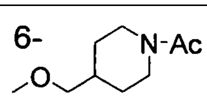 | Fum | F1:426, NMR2:2.00(3H, s), 7.23(1H, t, J=8.3Hz), 7.34(1H, s) |

(continued)

| | | | | | |
|---|---|---|---|---|---|
| 110 | 66 | - | 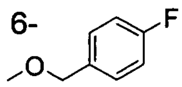 | Fum | F1:395, NMR2:5.13(2H, s), 6.53(1H, d, J=8.3Hz), 7.56-7.61(2H, m) |
| 111 | 66 | - | 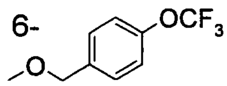 | Fum | F1:461, NMR2:5.19(2H, s), 6.69(1H, d, J=8.3Hz), 7.44(2H, d, J=7.9hz) |
| 112 | 66 | - | 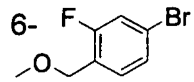 | Fum | F1:473, 475, NMR2:5.16(2H, s), 7.18(1H, s), 7.28(1H, t, J=8.3Hz) |
| 113 | 66 | - | 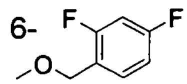 | 0.5 Fum | F1:413, NMR2:5.16(2H, s), 6.55(1H, d, J=8.3Hz), 7.68-7.75(1H, m) |
| 114 | 66 | - | 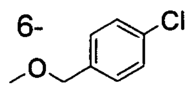 | Fum | F1:411, NMR2:5.14(2H, s), 7.24-7.29 (2H, m), 7.49-7.58(4H, m) |
| 115 | 66 | - | 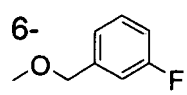 | Fum | F1:395, NMR2:1.23-1.34(1H, m), 6.54 (1H, d, J=7.3Hz), 7.45-7.50(1H, m) |
| 116 | 66 | 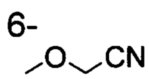 | H | Fum | E1:326, NMR2:5.23(2H, s), 6.63-6.67 (2H, m), 7.01(1H, s) |

[Table 19]

| | | | | | |
|---|---|---|---|---|---|
| 117 | 66 | 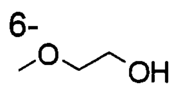 | H | 0.5 Fum | E1:331, NMR2:1.59-1.80(3H, m), 4.08 (2H, dd, J=4.9, 4.4Hz), 7.70(1H, s) |
| 118 | 66 | - | 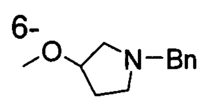 | Fum | E1:446, NMR2:4.92-5.06(1H, m), 7.16-7.32(6H, m), 7.49-7.50(1H, m) |
| 119 | 66 | - | 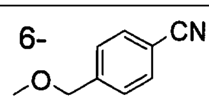 | Fum | F1:402, NMR2:5.25(2H, s), 7.21(1H, s), 7.92(2H, d, J=8.3Hz) |
| 120 | 66 | - | 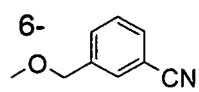 | 0.5 Fum | F1:402, NMR2:5.20(2H, s), 6.55(1H, d, J=8.3Hz), 8.00(1H, s) |
| 121 | 66 | - | 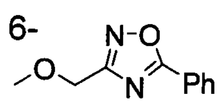 | Fum | F1:445, NMR2:1.43-1.62 (3H, m), 1.78-1.90 (1H, m), 7.31 (1H, t, J=8.4Hz) |
| 122 | 67 | 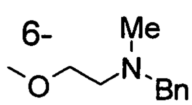 | H | Fum | E1:434, NMR2:2.22(3H, s), 6.50(1H, d, J=8.3Hz), 7.10(1H, s) |
| 123 | 67 | - | 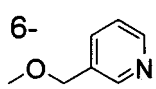 | 0.5 Fum | E1:378, NMR2:1.12-1.39(1H, m), 7.29 (1H, t, J=8.4Hz), 8.74(1H, s) |

(continued)

| | | | | | |
|---|---|---|---|---|---|
| 124 | 67 | - | 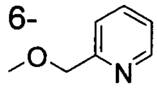 | Fum | E1:378, NMR2:1.30-1.43(1H, m), 7.26 (1H, t, J = 8.4 Hz), 7.67(1H, d, J=7.6Hz) |
| 125 | 67 | 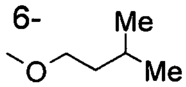 | H | 0.5 Fum | F1:357, NMR2:4.07(2H, t, J = 6.4 Hz), 6.49(1H, d, J=7.6Hz), 7.40(1H, s) |
| 126 | 67 | - | 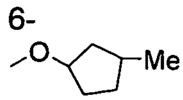 | 0.5 Fum | F1:369, NMR2:1.02(3H, d, J=6.4Hz), 7.20 (1H, t, J=8.2 Hz), 7.41(1H, d, J=2.8Hz) |
| 127 | 67 | - | 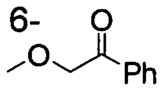 | 0.5 Fum | F1:405, NMR2:1.58-1.82(3H, m), 7.24 (1H, t, J=8.0Hz), 7.96(1H, s) |
| 128 | 71 | - | 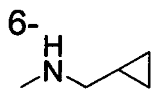 | 3HCl | F1:340, NMR2:0.19-0.23(2H, m), 2.95 (2H, d, J=6.4Hz), 7.11(1H, s) |
| 129 | 71 | - | 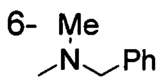 | 3HCl | F1:390, NMR2:2.72(3H, s), 6.86(1H, d, J=8.4Hz), 6.98(1H, d, J=8.4Hz) |
| 130 | 74 | - | 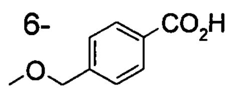 | 2HCl | F1:421, NMR2: 7.23(1H, s), 7.34(1H, t, J=8.3Hz), 7.97(2H, d, J=7.8Hz) |
| 131 | 74 | 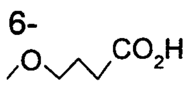 | H | 2HCl | F1:373, NMR2:2.78-2.94 (1H, m), 3.98-4.09 (2H, m), 7.23-7.33 (2H, m) |
| 132 | 74 | 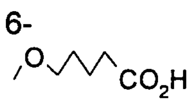 | H | 2HCl | F1:387, NMR2:2.80-2.95 (1H, m), 3.98-4.08 (2H, m), 7.24-7.34 (2H, m) |

[Table 20]

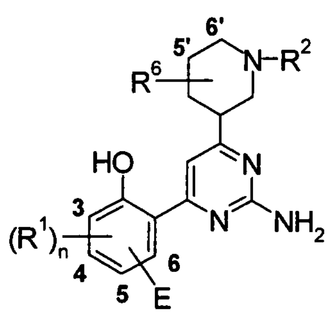

| Ex | Syn | $(R^1)_n$ | E | $R^2$ | $R^6$ | Sal | Dat |
|---|---|---|---|---|---|---|---|
| 77 | 77 | - | H | H | 6'-Me | 0.5 Fum | F1:285, NMR2:1.14(3H, d, J=8.4Hz), 6.82-6.96(2H, m), 7.19(1H, s) |
| 133 | 1 | 5-Me | H | H | $6'-(CH_2)_2Ph$ | 2HCl | E1:389, NMR2:2.65-2.82 (2H, m), 6.79-6.83(1H, m), 7.78-7.82 (1H, m) |

(continued)

| Ex | Syn | $(R^1)_n$ | E | $R^2$ | $R^6$ | Sal | Dat |
|---|---|---|---|---|---|---|---|
| 134 | 1 | 5-Me | H | $CH_2Ph$ | - | 2HCl | E1:375, NMR2:1.59-1.73(1H, m), 2.28(3H, s), 7.76(1H, s) |
| 135 | 1 | - | H | H | 5'-Me | 2HCl | F1:285, NMR2:0.96(3H, d, J=6.0Hz), 6.90-6.99(2H, m), 7.95-8.04(1H, m) |
| 136 | 1 | 5-Me | H | H | 5'-Me | 2HCl | F1:299, NMR2:0.96(3H, d, J=6.4Hz), 2.29(3H, s), 7.77-7.85(1H, m) |
| 137 | 1 | 5-Me | H | H | 6'-Me | 2HCl | F1:299, NMR2:1.29-1.36(3H, m), 7.19-7.27(1H, m), 7.78-7.86(1H, m) |
| 138 | 1 | 6-OH | H | H | 6'-Me | 2HCl | F1:301, NMR2:1.26-1.65(3H, m), 7.12-7.18(1H, m), 7.39-7.45(1H, m) |
| 139 | 4 | 6-OH | H | Boc | 6'-Me | - | F1:401, NMR2: 1.10-1.14(3H, m), 6.34-6.38(2H, m), 7.61-7.65(1H, m) |
| 140 | 66 | | 6- 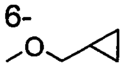 | H | 6'-Me | Fum | F1:355, NMR2:0.36-0.42(2H, m), 1.21(3H, d, J=6.4Hz), 7.64(1H, s) |

[Table 21]

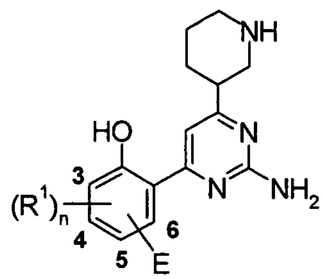

| Ex | Syn | $(R^1)n$ | E | Sal | Dat |
|---|---|---|---|---|---|
| 78 | 78 | 5-Et | H | 2HCl | E1:299, NMR2:1.20 (3H, t, J=7.6Hz), 6.92 (1H, d, J=8.3 Hz), 7.83 (1H, d, J=2.0Hz), $[\alpha]^{25}_{D}$= -30.0(c=0.3, EtOH) |
| 79 | 79 | 5-Et | H | 2HCl | E1:299, NMR2:1.20 (3H, t, J = 7.6Hz), 6.92 (1H, d, J=8.3 Hz), 7.83 (1H, d, J = 2.0 Hz), $[\alpha]^{25}_{D}$= +27.4(c=0.3, MeOH) |
| 141 | 78 | - | 6- 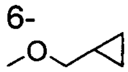 | 2HCl | F1:341, NMR2:2.81-2.93(1H, m), 6.57(1H, d, J=8.0Hz), 7.28 (3H, t, J=8.4Hz), $[\alpha]^{25}_{D}$= -7.59(c=0.3, MeOH) |
| 142 | 79 | - | 6- 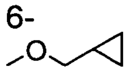 | 2HCl | F1:341, NMR2: 2.81-2.93(1H, m), 6.56(1H, d, J=8.4Hz), 7.29 (3H, t, J=8.4Hz), $[\alpha]^{25}_{D}$= +5.99(c=0.3, MeOH) |

[Table 22]

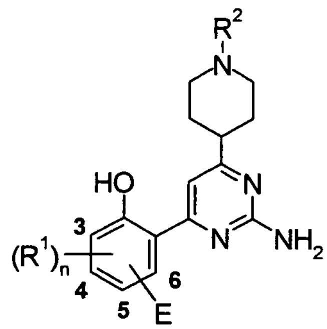

| Ex | Syn | $(R^1)n$ | E | $R_2$ | Sal | Dat |
|---|---|---|---|---|---|---|
| 143 | 1 | 6-OH | H | H | 2HCl | F1:341, NMR2:0.30-0.36(2H, m), 3.30-3.40 (2H, m), 6.57(1H, d, J=8.0Hz) |
| 144 | 4 | 6-OH | H | Boc | - | F1:387, NMR2:1.41(9H, s), 6.35(2H, d, J=8.0Hz), 7.61(1H, s) |
| 145 | 65 | - | 6- 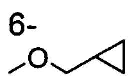 | H | 2HCl | F1:341, NMR2:0.30-0.36(2H, m), 3.30-3.40 (2H, m), 6.57(1H, d, J=8.0Hz) |
| 146 | 65 | 6- 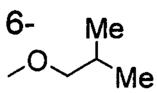 | H | H | 2HCl | F1:343, NMR2:0.90(6H, d, J=6.8Hz), 3.28-3.40(2H, m), 7.12(1H, s) |
| 147 | 65 | 6- 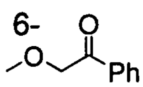 | H | H | 2HCl | F1:405, NMR2: 3.32-3.40(2H, m), 5.66(2H, s), 6.69(2H, d, J=8.4Hz) |

[Table 23]

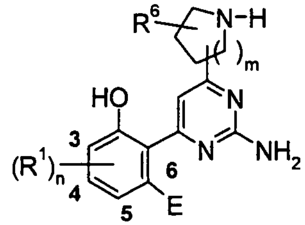

| Cmp | $(R^1)_n$ | E | 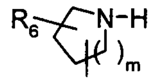 | Cmp | $(R^1)_n$ | E | 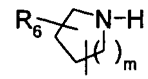 |
|---|---|---|---|---|---|---|---|
| 1 | H | 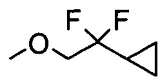 | 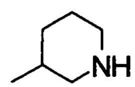 | 31 | H | 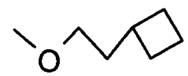 | 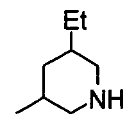 |
| 2 | 4-Me | | | 32 | 4-Me | | |
| 3 | H | 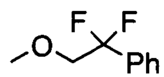 | | 33 | H | 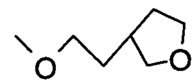 | |
| 4 | 5-Me | | | 34 | 5-Et | | |
| 5 | H | 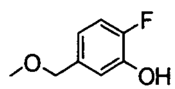 | | 35 | H | 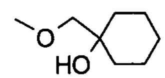 | 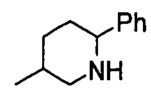 |
| 6 | 4-Et | | | 36 | 4-Me | | |
| 7 | H | 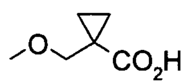 | | 37 | H | 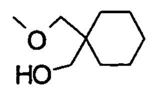 | |
| 8 | 5-Et | | | 38 | 5-Et | | |

(continued)

| Cmp | $(R^1)_n$ | E | 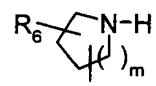 | Cmp | $(R^1)_n$ | E | 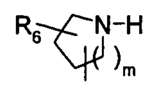 |
|---|---|---|---|---|---|---|---|
| 9 | H | 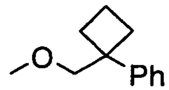 | | 39 | H | 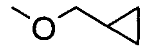 | 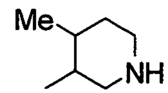 |
| 10 | 4-Me | | | 40 | 4-Me | | |
| 11 | 5-Et | | | 41 | H | 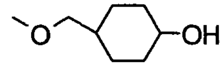 | |
| 12 | H | 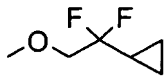 | 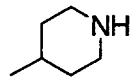 | 42 | 5-Et | | |
| 13 | 4-Me | | | 43 | H | 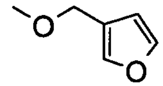 | 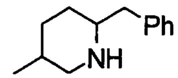 |
| 14 | H | 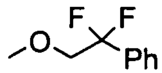 | | 44 | 4-Me | | |
| 15 | 5-Me | | | 45 | 5-Et | | |
| 16 | H | 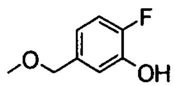 | | 46 | H | 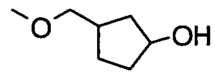 | 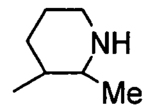 |
| 17 | 4-Et | | | 47 | 4-Me | | |
| 18 | H | 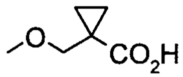 | | 48 | 5-Et | | |
| 19 | 5-Et | | | 49 | H | 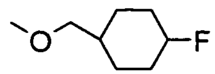 | 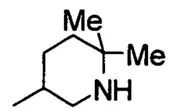 |
| 20 | H | 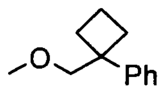 | | 50 | 4-Me | | |
| 21 | 4-Me | | | 51 | 5-Et | | |
| 22 | 5-Et | | | 52 | H | 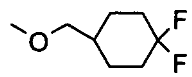 | 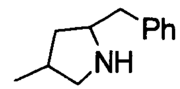 |
| 23 | H | 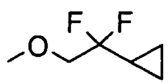 | | 53 | 4-Me | | |
| 24 | 4-Me | | | 54 | 5-Et | | |

(continued)

| Cmp | $(R^1)_n$ | E | 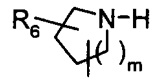 | Cmp | $(R^1)_n$ | E | 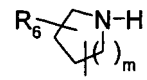 |
|---|---|---|---|---|---|---|---|
| 25 | H | 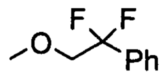 | 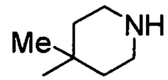 | 55 | H | 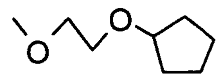 | 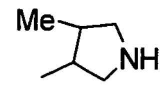 |
| 26 | 5-Me | | | 56 | 4-Me | | |
| 27 | H | 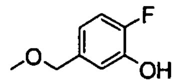 | | 57 | 5-Et | | |
| 28 | 4-Et | | | 58 | H | 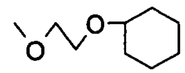 | 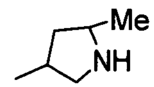 |
| 29 | H | 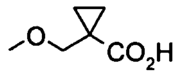 | | 59 | 4-Me | | |
| 30 | 5-Et | | | 60 | 5-Et | | |

INDUSTRIAL APPLICABILITY

The active ingredient of the medicine of the invention and the compound of the invention have an excellent antiinflammatory effect based on the IKK2-inhibitory effect thereof, and are therefore useful for remedies and preventives for inflammatory diseases and autoimmune diseases, especially for rheumatic diseases (rheumatoid arthritis, etc.), gastrointestinal disorders (ulcerative colitis, Crohn's disease), dermatologic disorders (atopic dermatitis, psoriasis, etc.), endcrine distorders (diabetes, etc.), neurologic disorders (multiple sclerosis, etc.), respiratory system diseases (asthma, etc.), and cancerous diseases, etc.

## Claims

**1.** An IKK2 inhibitor containing, as the active ingredient thereof, a 2-aminopyrimidine derivative of the following general formula (I) or its salt:

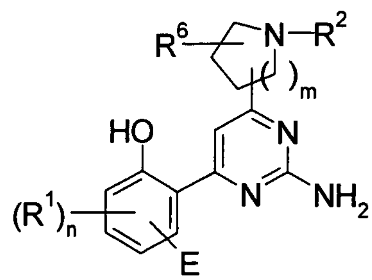

(I)

(wherein the symbols have the following meanings:

$R^1$: same or different from each other, each represents lower alkyl, -OH, -O-lower alkyl, halogen, halogeno lower alkyl, -S-$R^3$, -SO-$R^3$, -$SO_2$-$R^3$, -$NR^4$($R^5$), -$CO_2$-$R^3$, -CO-$NR^4$($R^5$), -$NR^4$-CO-$R^0$, -CN, -$NO_2$, -O-halogeno lower alkyl or lower alkenylene;
in these, lower alkyl and -O-lower alkyl may be substituted with one or two substituents selected from a group consisting of -O-$R^3$, -$NR^4$($R^5$), -CN and -$CO_2$-$R^3$;
$R^0$: lower alkyl;
$R^{00}$: lower alkylene;
$R^3$, $R^4$ and $R^5$: same or different from each other, these represent H or -$R^0$;
n: 0, 1 or 2;
E: H, -$E^1$ or -D-$E^1$;
$E^1$: optionally substituted cycloalkyl, optionally substituted phenyl, or optionally substituted monocyclic or bicyclic heterocyclic group;
D: bond, -O-, -S-, -$R^{00}$-, -O-$R^{00}$-, -$R^{00}$-O-, -$NR^4$-$R^{00}$-, -$R^{00}$-$NR^4$-, -$NR^4$-CO-, -CO-$NR^4$-, -O-$R^{00}$-$NR^4$-$R^{00}$-, -O-$R^{00}$-O-, -O-$R^{00}$-CO- or -O-$R^{00}$-CH(O-$R^3$)-;
$R^6$: H, -$R^{00}$-optionally substituted phenyl, or -$R^0$;
m: 0, 1, 2 or 3;
$R^2$: H, -$R^0$ or -Z-W;
Z: -$R^{00}$-, -CO-, -CO-$R^{00}$- or -$R^{00}$-CO-;
W: -O-$R^3$, -$NR^4$($R^5$), -$CR^{21}R^{22}R^{23}$, optionally substituted phenyl, or optionally substituted monocyclic or bicyclic heterocyclic group;
$R^{21}$: H or -$R^0$;
$R^{22}$: -$R^0$, -O-$R^3$ or -$NR^4R^5$);
$R^{23}$: -$R^0$ or optionally substituted phenyl).

**2.** A 2-aminopyrimidine derivative of the following general formula (I') or its salt:

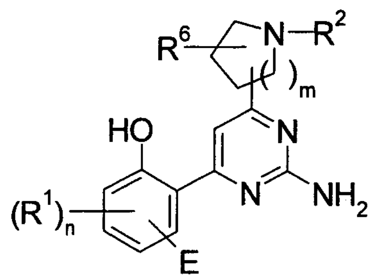

(I')

(wherein the symbols have the following meanings:

$R^1$: same or different from each other, each represents lower alkyl, -OH, -O-lower alkyl, halogen, halogeno lower alkyl, -S-$R^3$, -SO-$R^3$, -$SO_2$-$R^3$, -$NR^4(R^5)$, -$CO_2$-$R^3$, -CO-$NR^4(R^5)$, -$NR^4$-CO-$R^0$, -CN, -$NO_2$, -O-halogeno lower alkyl or lower alkenylene;
in these, lower alkyl and -O-lower alkyl may be substituted with one or two substituents selected from a group consisting of -O-$R^3$, -$NR^4(R^5)$, -CN and -$CO_2$-$R^3$;
$R^0$: lower alkyl;
$R^{00}$: lower alkylene;
$R^3$, $R^4$ and $R^5$: same or different from each other, these represent H or -$R^0$;
n: 0, 1 or 2;
E: H, -$E^1$ or -D-$E^1$;
$E^1$: optionally substituted cycloalkyl, optionally substituted phenyl, or optionally substituted monocyclic or bicyclic heterocyclic group;
D: bond, -O-, -S-, -$R^{00}$-, -O-$R^{00}$-, $R^{00}$-O-, -$NR^4$-$R^{00}$-, -$R^{00}$-$NR^4$-, -$NR^4$-CO-, -CO-$NR^4$-, -O-$R^{00}$-$NR^4$-$R^{00}$- -O-$R^{00}$-O-, -O-$R^{00}$-CO- or -O-$R^{00}$-CH(O-$R^3$)-;
$R^6$: H, -$R^{00}$-optionally substituted phenyl, or -$R^0$;
m: 0, 1, 2 or 3;
$R^2$: H, -$R^0$ or -Z-W;
Z: -$R^{00}$-, -CO-, -CO-$R^{00}$- or -$R^{00}$-CO-, provided that, when m is 1 or 2, then Z is- $R^{00}$-CO-;
W: -O-$R^3$, -$NR^4(R^5)$, -$CR^{21}R^{22}R^{23}$, optionally substituted phenyl, or optionally substituted monocyclic or bicyclic heterocyclic group;
$R^{21}$: H or -$R^0$;
$R^{22}$: -$R^0$, -O-$R^3$ or -$NR^4(R^5)$;
$R^{23}$: -$R^0$ or optionally substituted phenyl).

**3.** The derivative or its salt as claimed in claim 2, wherein $R^2$ is H.

**4.** The derivative or its salt as claimed in claim 3, wherein m is 1 or 2.

**5.** The derivative or its salt as claimed in claim 4, wherein E is H.

**6.** The derivative or its salt as claimed in claim 5, wherein $R^6$ is H.

**7.** A 2-aminopyrimidine derivative of the following general formula (I") or its salt:

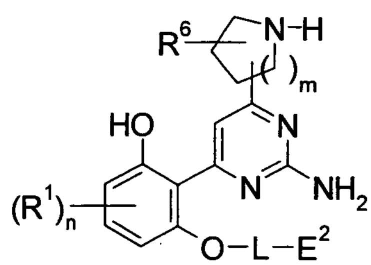

(I")

(wherein the symbols have the following meanings:

$R^1$: same or different from each other, each represents lower alkyl, -OH, -O-lower alkyl, halogen, halogeno lower alkyl, -S-$R^3$, -SO-$R^3$, -$SO_2$-$R^3$, -$NR^4(R^5)$, -$CO_2$-$R^3$, -CO-$NR^4(R^5)$, -$NR^4$-CO-$R^0$, -CN, -$NO_2$, -O-halogeno lower alkyl or lower alkenylene;
in these, lower alkyl and -O-lower alkyl may be substituted with one or two substituents selected from a group consisting of -O-$R^3$, -$NR^4(R^5)$, -CN and -$CO_2$-$R^3$;
$R^0$: lower alkyl;
$R^{00}$: lower alkylene;
$R^3$, $R^4$ and $R^5$: same or different from each other, these represent H or -$R^0$;
n: 0, 1 or 2;
$R^6$: H or -$R^0$;
m: 1 or 2;
L: bond, lower alkylene or lower alkylene-O-; in these, lower alkylene may be substituted with any of from 1 to 5 halogen, 1 or 2 -OH or oxo group;
$E^2$: optionally substituted cycloalkyl, optionally substituted phenyl, optionally substituted monocyclic heterocyclic group).

**8.** The derivative or its salt as claimed in claim 7, wherein $R^6$ is H.

**9.** The derivative or its salt as claimed in claim 2, which is selected from the following group: 2-(2-amino-6-piperidin-3-ylpyrimidin-4-yl)-4-methylphenol, 2-(2-amino-6-piperidin-3-ylpyrimidin-4-yl)-3-isobutoxyphenol, 2-(2-amino-6-piperidin-3-ylpyrimidin-4-yl)-3-(cyclobutylmethoxy)phenol, 2-(2-amino-6-piperidin-3-ylpyrimidin-4-yl)-3-(cyclopentylmethoxy)phenol, 2-(2-amino-6-piperidin-3-ylpyrimidin-4-yl)-3-[(4-bromobenzyl)oxy]phenol, (+)-2-(2-amino-6-piperidin-3-ylpyrimidin-4-yl)-4-ethylphenol, (-)-2-(2-amino-6-piperidin-3-ylpyrimidin-4-yl)-4-ethylphenol, 2-(2-amino-6-piperidin-3-ylpyrimidin-4-yl)-3-[(2-methylcyclopropyl)methoxy]phenol, 2-(2-amino-6-piperidin-3-ylpyrimidin-4-yl)-3-[(1-methylcyclopropyl)methoxy]phenol, 2-(2-amino-6-piperidin-3-ylpyrimidin-4-yl)-3-(2-phenoxypropoxy)phenol, 2-(2-amino-6-piperidin-3-ylpyrimidin-4-yl)-3-(2,2,3,3-tetrapropoxy)phenol, 2-(2-amino-6-piperidin-3-ylpyrimidin-4-yl)-3-(2-cyclopropylethoxy)phenol, (+)-2-(2-amino-6-piperidin-3-ylpyrimidin-4-yl)-3-(cyclopropylmethoxy)phenol, (-)-2-(2-amino-6-piperidin-3-ylpyrimidin-4-yl)-3-(cyclopropylmethoxy)phenol, 2-(2-amino-6-(6-methylpiperidin-3-yl)pyrimidin-4-yl)-3-(cyclopropylmethoxy)phenol, 2-(2-amino-6-piperidin-4-ylpyrimidin-4-yl)benzene-1,3-diol, 2-(2-amino-6-piperidin-4-ylpyrimidin-4-yl)-3-(cyclopropylmethoxy)phenol, 2-(2-amino-6-piperidin-4-ylpyrimidin-4-yl)-3-isobutoxyphenol and 2-[2-(2-amino-6-piperidin-4-ylpyrimidin-4-yl)-3-hydroxyphenoxy]-1-phenylethanone.

**10.** A pharmaceutical composition comprising a derivative or its salt stated in claim 2 and a pharmaceutically-acceptable carrier.

**11.** The pharmaceutical composition as claimed in claim 2, which is a remedy or preventive for inflammatory diseases or autoimmune diseases.

**12.** The pharmaceutical composition as claimed in claim 2, which is a remedy or preventive for rheumatoid arthritis.

**13.** Use of the derivative or its pharmaceutically-acceptable salt stated in claim 2, for production of remedies or preventives for rheumatoid arthritis.

**14.** A method for remedy or prevention of rheumatoid arthritis, which comprises administering a therapeutically-effective dose of a 2-aminopyrimidine derivative or its salt stated in claim 2 to patients.

**INTERNATIONAL SEARCH REPORT**

International application No.
PCT/JP2005/007178

A. CLASSIFICATION OF SUBJECT MATTER
Int.Cl[7] C07D401/04, A61K31/506, 31/55, A61P29/00, 35/00, 37/06, C07D401/14, 403/04, 413/14

According to International Patent Classification (IPC) or to both national classification and IPC

B. FIELDS SEARCHED

Minimum documentation searched (classification system followed by classification symbols)
Int.Cl[7] C07D401/04, A61K31/506, 31/55, A61P29/00, 35/00, 37/06, C07D401/14, 403/04, 413/14

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | JP 2003-95951 A (Sumitomo Pharmaceuticals Co., Ltd.),<br>03 April, 2003 (03.04.03),<br>Claims<br>(Family: none) | 1<br>2-13 |
| A | WO 02/02539 A1 (Abbott Laboratories),<br>10 January, 2002 (10.01.02),<br>Particularly, examples 14 to 35<br>& CA 2414461 A & AU 2001068718 A<br>& EP 1294704 A1 & JP 2004-502681 A<br>& NZ 523445 A | 1-13 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 July, 2005 (04.07.05) | 19 July, 2005 (19.07.05) |
| Name and mailing address of the ISA/<br>Japanese Patent Office<br>Facsimile No. | Authorized officer<br><br>Telephone No. |

Form PCT/ISA/210 (second sheet) (January 2004)

INTERNATIONAL SEARCH REPORT

International application No.
PCT/JP2005/007178

C (Continuation). DOCUMENTS CONSIDERED TO BE RELEVANT

| Category* | Citation of document, with indication, where appropriate, of the relevant passages | Relevant to claim No. |
|---|---|---|
| A | WO 96/32384 A1 (Taiho Pharmaceutical Co., Ltd.), 17 October, 1996 (17.10.96), Particularly, Claims & CA 2190973 A & AU 9652892 A1 & EP 767170 A1 & US 5786355 A & NO 9605334 A | 1-13 |
| A | JP 2001-139560 A (Dainippon Pharmaceutical Co., Ltd.), 22 May, 2001 (22.05.01), Full text (Family: none) | 1-13 |
| A | WO 02/096867 A2 (LG Biomedical Institute), 05 December, 2002 (05.12.02), Particularly, examples 79, 80, 82 & US 2003/187007 A1 & US 2003/208067 A1 & EP 1412327 A2 & JP 2004-534779 A | 1-13 |
| A | WO 04/26733 A1 (Merck Sharp and Dohme Ltd.), 24 November, 1994 (24.11.94), Full text & US 5763448 A & CA 2161702 A & AU 9466846 A1 & EP 698020 A1 & JP 8-509972 A | 1-13 |
| A | WO 02/44153 A1 (BAYER AG.), 06 June, 2002 (06.06.02), Full text & JP 2002-193938 A & JP 2004-514712 A & AU 2002/31628 A & EP 1339687 A1 & US 2004/097563 A1 & CA 2430354 A | 1-13 |
| A | WO 02/24679 A1 (BAYER AG.), 28 March, 2002 (28.03.02), Full text & JP 2002-114777 A & CA 2422923 A & AU 2001/089873 A & BR 2001/014073 A & EP 1326856 A1 & NZ 524811 A & US 6562811 B1 & BG 107632 A & ZA 2003/2226 A & NO 2003/1307 A | 1-13 |
| P,A | WO 2005/014556 A1 (Bayer Healthcare AG.), 17 February, 2005 (17.02.05), Particularly, example 12 & EP 1505064 A1 | 1-13 |
| P,A | WO 2004/080979 A1 (LG Life Science Ltd.), 23 September, 2004 (23.09.04), Particularly, Claim 3; compound 46 (Family: none) | 1-13 |

Form PCT/ISA/210 (continuation of second sheet) (January 2004)

INTERNATIONAL SEARCH REPORT

International application No.
PCT/JP2005/007178

**Box No. II Observations where certain claims were found unsearchable (Continuation of item 2 of first sheet)**

This international search report has not been established in respect of certain claims under Article 17(2)(a) for the following reasons:

1. [X] Claims Nos.: 14
because they relate to subject matter not required to be searched by this Authority, namely:
The invention as set forth in claim 14 is relevant to [methods for treatment of the human body by therapy]. (rule 39.1(iv) of the PCT)

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**
[ ] The additional search fees were accompanied by the applicant's protest.
[ ] No protest accompanied the payment of additional search fees.

Form PCT/ISA/210 (continuation of first sheet (2)) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02044153 A
- WO 02024679 A
- JP 2002114777 A
- US 5763448 A
- US 5948786 A
- JP 2003095951 A


### Non-patent literature cited in the description


- *Mol. Cell Biol.,* 1999, vol. 19, 4547-51
- *Annu. Rev. Immunol.,* 1994, vol. 12, 141-79
- *Nat. Rev. Drug Discov.,* 2004, vol. 3, 17-26
- *Science,* 1999, vol. 284, 321-5
- *Arthritis Rheum.,* 2001, vol. 44, 1897-907
- *Nat. Rev. Drug Discov.,* 2003, vol. 2, 473-88
- *Lancet,* 1999, vol. 354, 1932-9
- *Arthritis Rheum.,* 2001, vol. 43, 2648-2659
- *Drug Discovery Today,* 2002, vol. 7, 653-63
- *Prog. Med.,* 1985, vol. 5, 2157-2161
- Development of Medicines. Molecular Design. Hirokawa Publishing, 1990, vol. 7, 163-198
- **T. W. GREENE ; P. G. M. WUTS.** Protective Groups in Organic Synthesis. 1999
- Protective Groups in Organic Synthesis
- Jikken Kagaku Koza. Maruzen, 1992, vol. 22
- Courses in Experimental Chemistry. Maruzen, 1992, vol. 22
- Courses in Experimental Chemistry. Maruzen, 1992, vol. 20
- Courses in Experimental Chemistry. Maruzen, 1992, vol. 26
- Courses in Experimental Chemistry. 1992, vol. 20
- *Chem. Rev.,* 1995, vol. 95, 2457
- **SAMBROOK, J. et al.** Molecular Cloning-A Laboratory Manual. Cold Spring Harbor laboratory, 1989
- *The Japanese Journal of Pharmacology,* August 1997, vol. 74 (4), 313-22
- *The Japanese Journal of Pharmacology,* April 2002, vol. 88 (3), 332-340